# FASCICULE DE BREVET EUROPEEN

(11) **EP 0 606 382 B1**
(45) Date de publication et mention de la délivrance du brevet: **13.05.1998**
(21) Numéro de dépôt: 92921665.3
(22) Date de dépôt: 29.09.1992
(51) Int. Cl.: C07D 471/04, A61K 31/435

(54) **DERIVES DE BENZONAPHTYRIDINE-1,8 ET COMPOSITIONS ANTIMICROBIENNES**
1,8-BENZONAPHTHYRIDINDERIVATE UND ANTIMIKROBIELLE ZUSAMMENSETZUNGEN
1,8-BENZONAPHTHYRIDINE DERIVATIVES AND ANTIMICROBIAL COMPOSITIONS

(30) Priorité: 01.10.1991 FR 9112058
(43) Date de publication de la demande: 20.07.1994
(73) Titulaire: LABORATOIRE ROGER BELLON, 92201 Neuilly-sur-Seine (FR)
(72) Inventeur: BACQUE, Eric, F-91390 Morsang-sur-Orge (FR); BARREAU, Michel, F-91230 Montgeron (FR); DESCONCLOIS, Jean-François, F-75012 Paris (FR); GIRARD, Philippe 7, rue du Bois-Gaudron, F-91290 Arpajon (FR); KRYVENKO, Michel, F-75116 Paris (FR); LAVERGNE, Marc, Pierre, F-94520 Mandres-les-Roses (FR); PARIS, Jean-Marc, F-77360 Vaires-sur-Marne (FR); PICAUT, Guy, F-94800 Villejuif (FR)
(74) Mandataire: Savina, Jacques
(86) Numéro de dépôt international: FR9200901
(87) Numéro de publication internationale: WO9307144

(56) Documents cités:
- EP-A- 388 298
- EP-A- 431 991

## Description

La présente invention concerne de dérivés de la benzo[b] naphtyridine-1,8 de formule générale : 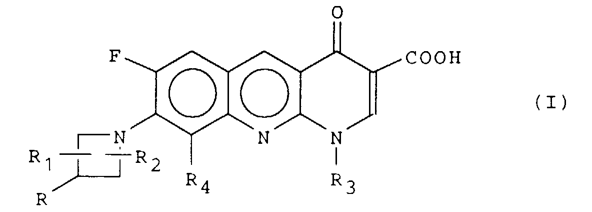 leurs sels, leur préparation et les compositions qui les contiennent.

Dans la demande de brevet EP 431 991 ont été décrits des dérivés de la benzonaphtyridine de structure : 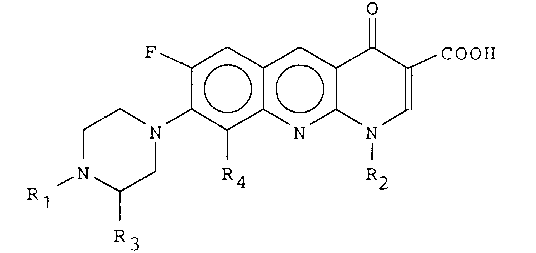 dans laquelle R1 est H, hydroxy ou alcoyle, R2 est H, alcoyle, fluoroalcoyle, cycloalcoyle, alcoyloxy ou alcoylamino, R3 est phényle ou phénylalcoyle éventuellement substitué, et R4 est H ou un atome de fluor. Ces produits sont utiles comme agents antimicrobiens.

Dans les demandes européennes EP 388 298 et EP 324 298 ont été décrites des azétidinyl oxo quinoléines ou oxo naphtyridines ayant une activité antimicrobienne.

Il a été trouvé que les produits de formule générale (I) dans laquelle :
- R représente un atome d'hydrogène ou un radical hydroxy, amino, alcoylamino dont la partie alcoyle est éventuellement substituée par un radical amino ou hydroxy ou représente un radical dialcoylamino dont les parties alcoyle peuvent éventuellement former avec l'atome d'azote auquel elles sont rattachées, un hétérocycle à 5 ou 6 chaînons contenant éventuellement un autre hétéroatome choisi parmi l'azote, l'oxygène ou le soufre, ou représente un radical cycloalcoylamino contenant 3 à 6 chaînons, ou un radical alcanoylamino, N-alcoyl N-alcanoyl amino ou aminoalcoylphénylamino,
- R₁ et R₂ identiques ou différents sont respectivement situés en position 2 et 3 et représentent des atomes d'hydrogène, des radicaux alcoyle, alcènyle contenant 2 à 4 atomes de carbone, phényle, phényle substitué par un atome d'halogène, ou par un radical alcoyle, alcoyloxy, hydroxy, nitro, amino, alcoylamino, dialcoylamino ou halogénoalcoyle, ou bien R₁ et R₂ sont situés en position 2 et représentent des radicaux alcoyle,
- R₃ représente un atome d'hydrogène ou un radical alcoyle, fluoroalcoyle, carboxyalcoyle, cycloalcoyle contenant 3 à 6 atomes de carbone, fluorophényle, difluorophényle, alcoyloxy ou alcoylamino, et
- R₄ représente un atome d'hydrogène ou un atome de fluor,
les radicaux alcoyle et alcanoyle cités ci-dessus étant droits ou ramifiés et contennant 1 à 4 atomes de carbone,
ainsi que leurs sels, et le cas échéant leurs stéréoisomères, manifestent une activité antibactérienne particulièrement intéressante.

Lorsque R représente un radical dialcoylamino dont les parties alcoyle forment avec l'atome d'azote, un hétérocycle, ce dernier peut être notamment pyrrolidinyle ou pipéridinyle.

Les produits de formule générale (I) peuvent exister à l'état de forme hydratée, il est entendu que ces hydrates entrent aussi dans le cadre de la présente invention.

Selon l'invention, les produits de formule générale (I) peuvent être obtenus par substitution d'une azétidine de formule générale : 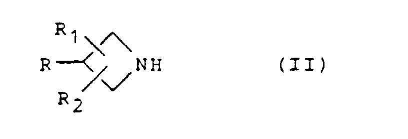 dans laquelle R, R₁ et R₂ sont définis comme précédemment, sur une benzo[b]naphtyridine-1,8 de formule générale : 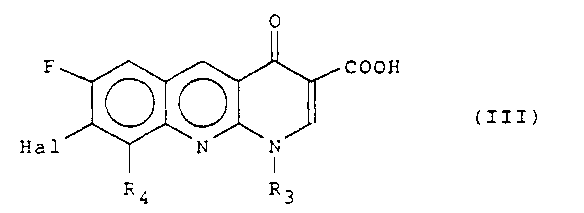 dans laquelle R₃ est défini comme précédemment, Hal est un atome de fluor, de chlore ou de brome si R₄ est hydrogène, ou bien Hal et R₄ sont simultanément des atomes de fluor.

L'action du dérivé de l'azétidine de formule générale (II) s'effectue généralement en présence d'un excès de ce dérivé comme accepteur d'acide dans des solvants organiques convenables. Il est possible d'opérer avec ou sans solvant, à une température comprise entre 20 et 150°C. Lorsque l'on opère en présence d'un solvant, la réaction s'effectue avantageusement dans des solvants tels que la pyridine, le diméthylformamide, le diméthylsulfoxyde ou l'acétonitrile. Il est également possible d'opérer en milieu aqueux.

Il peut être également avantageux d'opérer en présence d'un accepteur d'acide comme par exemple une base organique azotée (triéthylamine notamment), un carbonate alcalin (carbonate de sodium par exemple) ou un hydroxyde de métal alcalin ou alcalinoterreux.

Il est entendu que, dans le cas où le symbole R₃ du produit de formule générale (III) est un atome d'hydrogène, ou lorsque R est un radical amino, alcoylamino éventuellement substitué, cycloalcoylamino, ou aminoalcoylphénylamino, il est préférable de protéger préalablement le produit de départ. La protection et l'élimination du radical protecteur après la réaction, s'effectuent selon les méthodes habituelles.

La protection peut être réalisée par tout groupement compatible et dont la mise en oeuvre et l'élimination n'altère pas le reste de la molécule. Notamment, on opère selon les méthodes décrites par T.W. GREENE, Protective Groups in Organic Synthesis, A. Wiley-Interscience Publication (1981), ou par Mc OMIE, Protective Groups in Organic Chemistry, Plenum Press (1973).

A titre d'exemple, les groupements protecteurs peuvent être choisis parmi les radicaux triméthylsilyle, benzhydryle, tétrahydropyrannyle, formyle, acétyle, chloracétyle, trichloracétyle, trifluoroacétyle, éthoxycarbonyle, t.butoxycarbonyle, trichloréthoxycarbonyle.

Selon l'invention, les dérivés de benzo[b]naphtyridine-1,8 de formule générale (I) peuvent être aussi obtenus à partir de l'ester correspondant de formule générale : 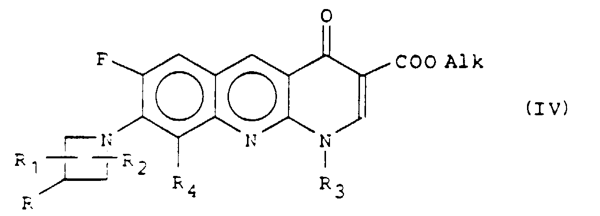 dans laquelle R₁, R₂ et R₄ sont définis comme précédemment, R est défini comme précédemment ou représente un radical amino protégé, R₃ est défini comme précédemment ou représente un radical alcoylamino protégé et Alk représente un radical alcoyle contenant 1 à 4 atomes de carbone en chaîne droite ou ramifiée, par toute méthode connue pour obtenir un acide à partir d'un ester sans toucher au reste de la molécule.

La préparation de l'acide à partir de l'ester s'effectue généralement par saponification en présence de potasse ou de soude, en milieu aqueux ou hydro-alcoolique, à une température comprise entre 20 et 100°C.

Dans les cas ou l'on hydrolyse un ester de formule générale (IV) pour lequel R est un radical alcanoylamino ou N-alcoyl N-alcanoylamino ou pour lequel R est un radical amino protégé, il est entendu que selon les conditions employées on obtient soit l'acide pour lequel R est un radical alcanoylamino ou N-alcoyl N-alcanoylamino ou pour lequel R est un radical amino protégé, soit l'acide pour lequel l'hydrolyse de l'amide a été effectuée simultanément c'est à dire pour lequel R est un radical amino. Les conditions opératoires sont choisies en fonction du produit final attendu. Lorsque R est un radical amino protégé il est bien entendu avantageux d'éliminer le radical protecteur simultanément.

Lorsque R₃ représente un radical alcoylamino protégé, le radical protecteur peut être tout groupement protecteur d'amino compatible avec la molécule. Il est particulièrement avantageux de choisir un radical protecteur qui peut être éliminé simultanément à l'hydrolyse de l'ester.

Le dérivé de la benzo[b]napthyridine-1,8 de formule générale (III) peut être obtenu à partir de l'ester correspondant de formule générale : 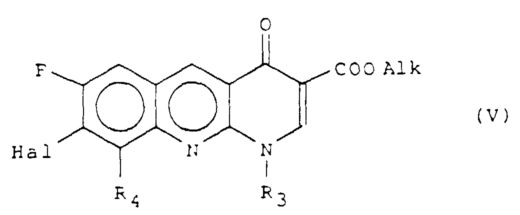 dans laquelle R₃, R₄, Hal et Alk sont définis comme précédemment, par application de la méthode décrite dans le brevet US 4 990 515 ou par analogie avec la technique décrite.

L'ester dérivé de la benzo[b]naphtyridine-1,8 de formule générale (V) peut être préparé par action de l'amino-3 triazine-1,2,4 (pour obtenir un produit pour lequel R₃ est un atome d'hydrogène) ou par action d'un produit de formule générale :

R₃ - NH₂ (VI)

dans laquelle R₃ est alcoyle, fluoroalcoyle, carboxyalcoyle cycloalcoyle, fluorophényle, difluorophényle, alcoyloxy ou alcoylamino éventuellement protégé sur un dérivé de la quinoléine de formule générale : 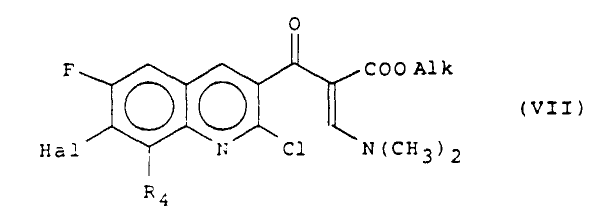 dans laquelle R₄, Hal et Alk sont définis comme précédemment, suivie de la cyclisation par action d'un agent accepteur d'acide.

Généralement, la réaction de l'amino-3 triazine-1,2,4 ou du produit de formule générale (VI) est mise en oeuvre dans un solvant organique comme un alcool (éthanol, méthanol par exemple) ou un solvant chloré (trichlorométhane par exemple), à une température comprise entre 10 et 25°C.

La cyclisation s'effectue dans un alcool à chaîne droite ou ramifiée contenant 1 à 4 atomes de carbone, à une température comprise entre 20°C et la température de reflux du mélange réactionnel.

L'agent accepteur d'acide peut être notamment choisi parmi les bases azotées (triéthylamine par exemple), le diaza-1,8-bicyclo[5.4.0]undécène-7, ou un excès de l'amine employée.

Les dérivés de la benzo[b]naphtyridine de formule générale (III) et (V) pour lesquels R₃ est un radical carboxyalcoyle, fluorophényle ou difluorophényle sont des produits nouveaux. Il est entendu que ces produits ainsi que leurs sels, lorsqu'ils existent, entrent aussi dans le cadre de la présente invention.

Le dérivé de la quinoléine de formule générale (VII) peut être obtenu comme décrit dans le brevet US 4 990 515.

Les dérivés des aminoazétidines de formule générale (II) peuvent être préparés selon les procédés décrits par : T. Okutani et Coll. Chem. Pharm. Bull., 22 (7) 1490 (1974); S. Chatterjee et Coll. Chem. Comm., 93 (1968); D. Nisato et Coll. J. Heterocyclic. Chem., 22, 961 (1985); Akira Morimoto et Coll., Chem,. Pharm. Bull., 21(1), 228 (1973); A.G. Anderson et Coll., J. Org. Chem., 37, 3953 (1972); V.R. Gaertner., J. Org. Chem., 2972 (1967), J.N. Wells et Coll., J. Org. Chem., 34, 1477 (1969), J. Antibiotics, 39 (9), 1243 (1986) et J. Pharm. Soc., 60(1), 156 (1971); EP 406 112; EP 314 362; EP 106 489; EP 324 298; JP 74 109 369 [C.A.83-9760 (1975)]; US 4 834 846 ou par analogie avec ces méthodes.

L'amino-3 phényl-3 azétidine peut être obtenue par réduction de l'azétidinone-2 correspondante, selon la méthode décrite dans J. Pharm. Sci., 60, 5, (1971). L'amino-3 phényl-3 azétidinone-2 est préparée par analogie avec la méthode décrite dans J. Am. Chem. Soc., 111, 1073 (1989) puis libération du radical protecteur de l'amine.

Le dérivé de benzo[b]naphtyridine-1,8 de formule générale (IV) peut être obtenu à partir de la benzo[b]naphtyridine de formule générale (V), par substitution d'un dérivé de l'azétidine de formule générale (II).

on opère avantageusement dans les conditions décrites précédemment pour obtenir un produit de formule générale (I) à partir d'une azétidine de formule générale (II) et d'une benzo[b]naphtyridine-1,8 de formule générale (III). Il est entendu que dans l'alternative ou l'on opère en milieux aqueux on peut obtenir directement le produit de formule générale (I) sans isoler intermédiarement le dérivé de formule générale (IV).

Selon l'invention, le cas échéant, lorsque l'on veut obtenir les stéréoisomères des dérivés de la benzonaphtyridine de formule générale (I), on effectue la séparation des formes stéréoisoméres des azétidines de formule générale (II) par toute méthode connue et compatible avec la molécule. A titre d'exemple, la séparation s'effectue par acylation au moyen d'un acide ou d'un dérivé réactif d'un acide chiral, séparation des isomères par chromatographie liquide hautes performances, puis désacylation selon la méthode décrite par P. G. Gas-seman et coll., J. Am. Chem. Soc., 98 (5), 1275 (1976). Il est également possible d'effectuer la séparation des stéréoisomères par chromatographie liquide hautes performances sur phase chirale.

Les nouveaux produits selon la présente invention ainsi que leurs intermédiaires de synthèse peuvent être éventuellement purifiés par des méthodes physiques telles que la cristallisation ou la chromatographie.

Les produits selon la présente invention ainsi que leurs intermédiaires de formule générale (III) et le cas échéant leurs intermédiaires de formule générale (V) peuvent être transformés en sels métalliques ou en sels d'addition avec les bases azotées selon les méthodes connues en soi. Ces sels peuvent être obtenus par action d'une base métallique (par exemple alcaline ou alcalino-terreuse), de l'ammoniac ou d'une amine sur un produit selon l'invention dans un solvant approprié tel qu'un alcool, un éther ou l'eau ou par réaction d'échange avec un sel d'un acide organique. Le sel formé précipite après concentration éventuelle de sa solution, il est séparé par filtration, décantation ou lyophilisation.

Les nouveaux produits selon l'invention peuvent être également transformés en sels d'addition avec les acides. Les produits de formule générale (I) obtenus sous forme de ces sels, peuvent être libérés et transformés en sels d'autres acides selon les méthodes habituelles.

Comme exemples de sels pharmaceutiquement acceptables, peuvent être cités les sels avec les métaux alcalins (sodium, potassium, lithium) ou avec les métaux alcalino-terreux (magnésium, calcium), le sel d'ammonium, les sels de bases azotées (éthanolamine, diéthanolamine, triméthylamine, triéthylamine, méthylamine, propylamine, diisopropylamine, NN-diméthyléthanolamine, benzylamine, dicyclohexylamine, N-benzyl phénéthylamine, NN'-dibenzyléthylènediamine, diphénylènediamine, benzhydrylamine, quinine, choline, arginine, lysine, leucine, dibenzylamine), ainsi que les sels d'addition avec des acides minéraux (chlorhydrates, bromhydrates, sulfates, nitrates, phosphates) ou organiques (succinates, fumarates, maléates, méthanesulfonates, p.toluènesulfonates, iséthionates).

Les nouveaux dérivés de benzo[b]naphtyridine-1,8 de formule générale (I) selon la présente invention et leurs sels pharmaceutiquement acceptables présentent des propriétés antibactériennes particulièrement intéressantes. Ils manifestent une activité remarquable in vitro et in vivo sur les germes gram-positifs et d'une manière générale sur les germes responsables de la plupart des infections des voies aériennes hautes et basses. De plus les nouveaux dérivés de benzo[b]naphtyridine-1,8 de formule générale (I) manifestent une activité antibactérienne particulièrement intéressante sur les germes gram-négatifs.

In vitro, les produits de formule générale (I) se sont montrés actifs à une concentration comprise entre 0,06 et 4 µg/cm³ sur staphylococcus aureus IP 8203 et à une concentration comprise entre 0,25 et 20 µg/cm³ sur Escherichia coli souche NIHJ JC2.

In vivo, les produits de formule générale (I) se sont montrés actifs sur les infections expérimentales de la souris à staphylococcus aureus IP 8203 à des doses comprises entre 2 et 200 mg/kg par voie orale.

Par ailleurs certains des produits selon l'invention se sont révélés particulièrement intéressants sur mycoplasma. Leur concentration minimale inhibitrice est comprise entre 0,03 et 8 µg/ml.

Enfin les produits selon l'invention ne présentent pas de toxicité au doses utilisées. Ils sont généralement atoxiques à 500 mg/kg par voie orale chez la souris.

Les exemples suivants donnés à titre non limitatif illustrent la présente invention.

### EXEMPLE 1

Acide (amino-3 azétidinyl-1)-8 fluoro-7 méthyl-1 oxo-4 dihydro-1,4 benzo[b]naphtyridine-1,8 carboxylique-3 ;

Une suspension de 1,16 g d'acide difluoro-7,8 méthyl-1 oxo-4 dihydro-1,4 benzo[b]naphtyridine-1,8 carboxylique-3 et de 1,38 g d'amino-3 azétidine dans 15 cm³ de diméthylsulfoxyde est chauffée, sous agitation, à une température voisine de 95°C pendant 6 heures. Après refroidissement environ 20°C, on ajoute au mélange réactionnel 100 cm³ d'eau. L'insoluble est éssoré, lavé par 3 fois 20 cm³ d'eau repris par 100 cm³ d'eau et additionné de 4 cm³ d'acide méthanesulfonique N. Après élimination d'un léger insoluble par filtration sur silice diatomée et addition de 4 cm³ de soude aqueuse N, la suspension obtenue est concentrée sous pression réduite (20 kPa) à une température voisine de 60°C, à un volume d'environ 80 cm³. L'insoluble est essoré, lavé par 100 cm³ d'eau, 100 cm³ d'éthanol et recristallisé dans 150 cm³ de diméthylformamide. On obtient 0,7 g d'acide (amino-3 azétidinyl-1)-8 fluoro-7 méthyl-1 oxo-4 dihydro-1,4 benzo[b]naphtyridine-1,8 carboxylique-3 sous forme d'un solide jaune se décomposant à 358°C.

L'acide difluoro-7,8 méthyl-1 oxo-4 dihydro-1,4 benzo[b]naphtyridine-1,8 carboxylique-3 est préparé comme décrit dans le brevet US 4 990 515.

L'amino-3 azétidine a été préparée selon la méthode décrite par Dino Nisato et coll., J. Het. Chem.,22, 961, (1985).

### EXEMPLE 2

Acide (diméthylamino-3 azétidinyl-1)-8 fluoro-7 méthyl-1 oxo-4 dihydro-1,4 benzo[b]naphtyridine-1,8 carboxylique-3 :

Une suspension de 1,9 g de (diméthylamino-3 azétidinyl-1)-8 éthoxycarbonyl-3 fluoro-7 méthyl-1 oxo-4 dihydro-1,4 benzo[b]naphtyridine-1,8 dans 20 cm³ d'éthanol et 19 cm³ de potasse aqueuse 0,5 N est chauffée, sous agitation, à une température voisine de 80°C pendant 5 heures. Après refroidissement à environ 5°C, le mélange réactionnel est additionné de 9,5 cm³ d'une solution aqueuse d'acide méthanesulfonique N. L'insoluble est essoré, lavé par 2 fois 10 cm³ d'eau, 3 fois 25 cm³ d'éthanol et recristallisé dans 125 cm³ de diméthylformamide. On obtient 1,4 g d'acide (diméthylamino-3 azétidinyl-1)-8 fluoro-7 méthyl-1 oxo-4 dihydro-1,4 benzo[b]naphtyridine-1,8 carboxylique-3 sous forme d'un solide jaune se décomposant à 312°C.

La (diméthylamino-3 azétidinyl-1)-8 éthoxycarbonyl-3 fluoro-7 méthyl-1 oxo-4 dihydro-1,4 benzo[b]naphtyridine-1,8 a été préparée de la manière suivante:

Une suspension de 2 g d'éthoxycarbonyl-3 difluoro-7,8 méthyl-1 oxo-4 dihydro-1,4 benzo[b]naphtyridine-1,8, de 1,2 g de dichlorhydrate de diméthylamino-3 azétidine et de 1,5 g de carbonate de sodium dans 30 cm³ de diméthylsulfoxyde est chauffée, sous agitation à une température voisine de 95°C pendant 5 heures. Après refroidissement à environ 20°C, le mélange réactionnel est additionné de 60 cm³ d'eau. L'insoluble est essoré et lavé par 3 fois 20 cm³ d'eau. On obtient 2 g de (diméthylamino-3 azétidinyl-1)-8 éthoxycarbonyl-3 fluoro-7 méthyl-1 oxo-4 dihydro-1,4 benzo[b]naphtyridine-1,8 sous forme d'un solide jaune fondant à 224°C qui est utilisé sans autre purification pour les étapes ultérieures.

L'éthoxycarbonyl-3 difluoro-7,8 méthyl-1 oxo-4 dihydro-1,4 benzo[b]naphtyridine-1,8 peut être préparé comme décrit dans le brevet US 4 970 213.

### EXEMPLE 3

Acide (amino-3 azétidinyl-1)-8 éthyl-1 fluoro-7 oxo-4 dihydro-1,4 benzo[b]naphtyridine-1,8 carboxylique-3 :

On opère dans les conditions décrites à l'exemple 2, mais à partir de 0,72 g d'(amino-3 azétidinyl-1)-8 éthoxycarbonyl-3 éthyl-1 fluoro-7 oxo-4 dihydro-1,4 benzo[b]naphtyridine-1,8. On obtient, sans recristallisation, 0,6 g d'acide (amino-3 azétidinyl-1)-8 éthyl-1 fluoro-7 oxo-4 dihydro-1,4 benzo[b]naphtyridine-1,8 carboxylique-3 monohydraté, sous forme d'un solide jaune se décomposant à 306°C.

L'(amino-3 azétidinyl-1)-8 éthoxycarbonyl-3 éthyl-1 fluoro-7 oxo-4 dihydro-1,4 benzo[b]naphtyridine-1,8 a été préparée dans les conditions décrites à l'exemple 2, mais à partir de 1,7 g d'éthoxycarbonyl-3 éthyl-1 difluoro-7,8 oxo-4 dihydro-1,4 benzo[b]naphtyridine-1,8 et de 1,62 g de diméthanesulfonate d'amino-3 azétidine. Le produit brut est repris par 100 cm³ de diméthylformamide et agité pendant 10 minutes à environ 150°C. Après refroidissement à environ 20°C, on élimine un insoluble par filtration. Le filtrat est concentré à sec, sous pression réduite (20 kPa) à environ 60°C. Le résidu est recristallisé dans 50 cm³ d'éthanol. On obtient 0,72 g d'(amino-3 azétidinyl-1)-8 éthoxy-carbonyl-3 éthyl-1 fluoro-7 oxo-4 dihydro-1,4 benzo[b]naphtyri-dine-1,8 sous forme d'un solide jaune fondant à 255-256°C.

L'éthoxycarbonyl-3 éthyl-1 difluoro-7,8 oxo-4 dihydro-1,4 benzo[b] naphtyridine-1,8 a été préparée comme décrit dans le brevet US 4 970 213.

### EXEMPLE 4

Acide (diméthylamino-3 azétidinyl-1)-8 éthyl-1 fluoro-7 oxo-4 dihydro-1,4 benzo[b]naphtyridine-1,8 carboxylique-3 :

On opère dans les conditions décrites à l'exemple 2, mais à partir de 2 g de (diméthylamino-3 azétidinyl-1)-8 éthoxycarbonyl-3 éthyl-1 fluoro-7 oxo-4 dihydro-1,4 benzo[b]naphtyridine-1,8. On obtient 1,68 g d'acide (diméthylamino-3 azétidinyl-1)-8 éthyl-1 fluoro-7 oxo-4 dihydro-1,4 benzo[b]naphtyridine-1,8 carboxylique-3 sous forme d'un solide jaune se décomposant à 278°C.

La (diméthylamino-3 azétidinyl-1)-8 éthoxycarbonyl-3 éthyl-1 fluoro-7 oxo-4 dihydro-1,4 benzo[b]naphtyridine-1,8 a été préparée dans les conditions décrites à l'exemple 2, mais à partir de 1,7 g d'éthoxycarbonyl-3 éthyl-1 difluoro-7,8 oxo-4 dihydro-1,4 benzo[b] naphtyridine-1,8 et de 1,3 g de dichlorhydrate de diméthylamino-3 azétidine. Après réaction, le mélange réactionnel, refroidi à environ 20°C est versé dans 50 cm³ d'eau et extrait par 3 fois 100 cm³ de dichlorométhane. Les extraits organiques réunis sont lavés par 3 fois 150 cm³ d'eau et séchés sur sulfate de magnésium. Après concentration à sec sous pression réduite (20 kPa) à environ 40°C, le résidu est repris par 50 cm³ d'éther éthylique, filtré et lavé par 2 fois par 50 cm³ du même solvant. On obtient 2 g de (diméthylamino-3 azéti-dinyl-1)-8 éthoxycarbonyl-3 éthyl-1 fluoro-7 oxo-4 dihydro-1,4 benzo[b]naphtyridine-1,8 sous forme d'un solide jaune fondant à 232°C qui est utilisé sans autre purification pour les étapes ultérieures.

### EXEMPLE 5

Acide (amino-3 azétidinyl)-8 cyclopropyl-1 fluoro-7 oxo-4 dihydro-1,4 benzo[b]naphtyridine-1,8 carboxylique-3 :

On opère dans les conditions décrites à l'exemple 2, mais à partir de 1 g d'(amino-3 azétidinyl-1)-8 cyclopropyl-1 éthoxycarbonyl-3 fluoro-7 oxo-4 dihydro-1,4 benzo[b]naphtyridine-1,8. On obtient 0,56 g d'acide (amino-3 azétidinyl-1)-8 cyclopropyl-1 fluoro-7 oxo-4 dihydro-1,4 benzo[b]naphtyridine-1,8 carboxylique-3 hémihydrate, sous forme d'un solide jaune se décomposant à 298-303°C,

L'(amino-3 azétidinyl-1)-8 cyclopropyl-1 éthoxycarbonyl-3 fluoro-7 oxo-4 dihydro-1,4 benzo[b]naphtyridine-1,8 a été préparée dans les conditions de l'exemple 2, mais à partir de 1,7 g de cyclopropyl-1 éthoxycarbonyl-3 difluoro-7,8 oxo-4 dihydro-1,4 benzo[b]naphtyridine-1,8 et de 1,88 g de diméthanesulfonate d'amino-3 azétidine. Après 1 recristallisation dans 50 cm³ d'éthanol, on obtient 1,05 g d'(amino-3 azétidinyl-1)-8 cyclopropyl-1 éthoxycarbonyl-3 fluoro-7 oxo-4 dihydro-1,4 benzo[b]naphtyridine-1,8 sous forme d'un solide jaune fondant à 178-180°C.

La cyclopropyl-1 éthoxycarbonyl-3 difluoro-7,8 oxo-4 dihydro-1,4 benzo[b]naphtyridine-1,8 peut être préparée comme décrit dans le brevet US 4 970 213.

### EXEMPLE 6

Acide cyclopropyl-1 (diméthylamino-3 azétidinyl-1)-8 fluoro-7 oxo-4 dihydro-1,4 benzo[b]naphtyridine-1,8 carboxylique-3 :

On opère dans les conditions décrites à l'exemple 4, mais à partir de 1,27 g de cyclopropyl-1 (diméthylamino-3 azétidinyl-1)-8 éthoxycarbonyl-3 fluoro-7 oxo-4 dihydro-1,4 benzo[b]naphtyridine-1,8. On obtient 0,8 g d'acide cyclopropyl-1 (diméthylamino-3 azétidinyl-1)-8 fluoro-7 oxo-4 dihydro-1,4 benzo[b]naphtyridine-1,8 carboxylique-3 sous forme d'un solide jaune fondant à 264°C.

La cyclopropyl-1 (diméthylamino-3 azétidinyl-1)-8 éthoxycarbonyl-3 fluoro-7 oxo-4 dihydro-1,4 benzo[b]naphtyridine-1,8 a été préparée dans les conditions décrites à l'exemple 4, mais à partir de 1,4 g de cyclopropyl-1 éthoxycarbonyl-3 difluoro-7,8 oxo-4 dihydro-1,4 benzo[b]naphtyridine-1,8 et de 1,04 g de dichlorhydrate de diméthylamino-3 azétidine. Après concentration à sec des extraits organiques réunis, le solide obtenu est recristallisé dans 40 cm³ d'éthanol. On obtient 1,2 g de cyclopropyl-1 (diméthylamino-3 azétidinyl-1)-8 éthoxycarbonyl-3 fluoro-7 oxo-4 dihydro-1,4 benzo[b]naphtyridine-1,8 sous forme d'un solide jaune fondant à 225°C.

### EXEMPLE 7

Acide (amino-3 azétidinyl-1)-8 cyclopropyl-1 difluoro-7,9 oxo-4 dihydro-1,4 benzo[b]naphtyridine-1,8 carboxylique-3 :

On opère dans les conditions décrites à l'exemple 2, mais à partir de 0,8 g d'(amino-3 azétidinyl-1)-8 cyclopropyl-1 éthoxycarbonyl-3 difluoro-7,9 oxo-4 dihydro-1,4 benzo[b]naphtyridine-1,8. On obtient sans recristallisation 0,6 g d'acide (amino-3 azétidinyl-1)-8 cyclopropyl-1 difluoro-7,9 oxo-4 dihydro-1,4 benzo[b]naphtyridine-1,8 carboxylique-3 monohydraté, sous forme d'un solide jaune se décomposant à 308-312°C.

L'(amino-3 azétidinyl-1)-8 cyclopropyl-1 éthoxycarbonyl-3 difluoro-7,9 oxo-4 dihydro-1,4 benzo[b]naphtyridine-1,8 a été préparée dans les conditions de l'exemple 2, mais à partir de 1,3 g de cyclopropyl-1 éthoxycarbonyl-3 trifluoro-7,8,9 oxo-4 dihydro-1,4 benzo[b]naphtyridine-1,8 et de 1,32 g de diméthanesulfonate d'amino-3 azétidine. Après recristallisation dans 50 cm³ d'éthanol, on obtient 0,8 g d'(amino-3 azétidinyl-1)-8 cyclopropyl-1 éthoxycarbonyl-3 difluoro-7,9 oxo-4 dihydro-1,4 benzo[b]naphtyridine-1,8 sous forme d'un solide jaune fondant à 236-238°C.

La cyclopropyl-1 éthoxycarbonyl-3 trifluoro-7,8,9 oxo-4 dihydro-1,4 benzo[b]naphtyridine-1,8 a été préparée comme décrit dans le brevet US 4 970 213.

### EXEMPLE 8

L'acide (diéthylamino-3 azétidinyl-1)-8 fluoro-7 méthyl-1 oxo-4 dihydro-1,4 benzo[b]naphtyridine-1,8 carboxylique-3 a été préparé dans les conditions de l'exemple 2, mais à partir de 1,75 g de (diéthylamino-3 azétidinyl-1)-8 éthoxycarbonyl-3 fluoro-7 méthyl-1 oxo-4 dihydro-1,4 benzo[b]naphtyridine-1,8. On obtient 1,4 g d'acide (diéthylamino-3 azétidinyl-1)-8 fluoro-7 méthyl-1 oxo-4 dihydro-1,4 benzo[b]naphtyridine-1,8 carboxylique-3 sous forme d'un solide jaune se décomposant à 297°C.

La (diéthylamino-3 azétidinyl-1)-8 éthoxycarbonyl-3 fluoro-7 méthyl-1 oxo-4 dihydro-1,4 benzo[b]naphtyridine-1,8 a été préparée dans les conditions de l'exemple 2, mais à partir de 2 g d'éthoxycarbonyl-3 difluoro-7,8 méthyl-1 oxo-4 dihydro-1,4 benzo[b]naphtyridine-1,8 et de 1,4 g de dichlorhydrate de diéthylamino-3 azétidine. Après recristallisation du produit brut dans 500 cm3 de méthanol, on obtient 2,07 g de (diéthylamino-3 azétidinyl-1)-8 éthoxycarbonyl-3 fluoro-7 méthyl-1 oxo-4 dihydro-1,4 benzo[b]naphtyridine-1,8 sous forme d'un solide jaune fondant à 260°C.

La diéthylamino-3 azétidine a été préparée sous forme de dichlorhydrate fondant à 175°C selon la méthode décrite dans la demande de brevet japonais 74 109 369.

### EXEMPLE 9

Acide (éthylamino-3 azétidinyl-1)-8 fluoro-7 méthyl-1 oxo-4 dihydro-1,4 benzo[b]naphtyridine-1,8 carboxylique-3:

On opère dans les conditions décrites à l'exemple 2, mais à partir de 0,51 g d'éthoxycarbonl-3 (éthylamino-3 azétidinyl-1)-8 fluoro-7 méthyl-1 oxo-4 dihydro-1,4 benzo[b]naphtyridine-1,8. On obtient 0,4 g d'acide (éthylamino-3 azétidinyl-1)-8 fluoro-7 méthyl-1 oxo-4 dihydro-1,4 benzo[b]naphtyridine-1,8 carboxylique-3 monohydrate sous forme d'un solide jaune se décomposant à 270°C.

L'éthoxycarbonyl-3 (éthylamino-3 azétidinyl-1)-8 fluoro-7 méthyl-1 oxo-4 dihydro-1,4 benzo[b]naphtyridine-1,8 a été préparé dans les conditions de l'exemple 2, mais à partir de 2 g de éthoxycarbonyl-3 difluoro-7,8 méthyl-1 oxo-4 dihydro-1,4 benzo[b]naphtyridine-1,8 et de 1,3 g de dichlorhydrate d'éthylamino-3 azétidine. Après addition de 300 cm³ d'eau au mélange réactionnel, la suspension obtenue est extraite par 3 fois 100 cm³ de dichlorométhane. Les extraits organiques réunis sont séchés sur sulfate de magnésium et concentrés à sec sous pression réduite (20 kPa) à une température voisine de 40°C. Le produit obtenu est purifié par chromatographie sur gel de silice 0,063-0,200 mm en suspension dans le dichlorométhane à 1 % de méthanol. Le produit cherché est élué par 500 cm³ de dichlorométhane à 5 % de méthanol. On obtient 0,51 g d'éthoxycarbonyl-3 (éthylamino-3 azétidinyl-1)-8 fluoro-7 méthyl-1 oxo-4 dihydro-1,4 benzo[b]naphtyridine-1,8 sous forme d'un solide jaune se décomposant à 215°C qui est utilisé sans autre purification pour les étapes ultérieures.

Le dichlorhydrate de l'éthylamino-3 azétidine peut être préparé par analogie avec la méthode décrite par Dino Nisato et Coll., J. Het. Chem., 22, 961 (1985) :

4 g de dichlorhydrate de benzhydryl-1 éthylamino-3 azétidine dans 50 cm3 de méthanol sont hydrogénés à pression atmosphérique, à une température voisine de 20°C, pendant 1 heure en présence de 800 mg d'hydroxyde de palladium à 20 % sur charbon. Après élimination du catalyseur par filtration, concentration à sec sous pression réduite (20 kPa) à environ 40°C, le résidu est repris par 30 cm³ d'éther éthylique, essoré, lavé par 3 fois 20 cm³ du même solvant. On obtient 2,05 g de dichlorhydrate d'éthylamino-3 azétidine sous forme d'un solide incolore fondant à 200°C, qui est utilisé, sans autre purification pour les étapes ultérieures.

Le dichlorhydrate de benzhydryl-1 éthylamino-3 azétidine est préparé de la manière suivante:

Un mélange de 55 g de benzhydryl-1 méthanesulfonyloxy-3 azétidine et de 70 g d'éthylamine dans 400 cm³ de méthanol est chauffé pendant 16 heures à environ 60°C. Le mélange réactionnel est ensuite concentré à sec sous pression réduite (20 kPa) à environ 40°C et le résidu repris par 200 cm³ d'éther éthylique. La phase organique lavée par 90 cm³ de soude aqueuse 2 N et 3 fois 30 cm³ d'eau est séchée sur sulfate de sodium et concentrée à sec sous pression réduite (20 kPa), à environ 40°C, on ajoute à l'extrait sec obtenu, 22,6 cm³ d'acide chlorhydrique 12 N, concentre de nouveau à sec, dans les conditions précédentes, mais à environ 70°C. Le résidu est repris par 100 cm³ d'acétate d'éthyle et 200 cm³ d'acétone et essoré. On obtient 41,4 g de dichlorhydrate de benzhydryl-1 éthylamino-3 azétidine sous forme d'un solide incolore fondant à 180°C utilisé sans autre purification pour les étapes ultérieures.

La benzhydryl-1 méthanesulfonyloxy-3 azétidine a été préparée selon la méthode décrite par A. G. Anderson et Coll., J. Org. Chem. 37, 3953 (1972).

### EXEMPLE 10

Acide (amino-3 azétidinyl-1)-8 fluoro-7 méthylamino-1 oxo-4 dihydro-1,4 benzo[b]naphtyridine-1,8 carboxylique-3.

Une suspension de 1,8 g d'(acétylamino-3 azétidinyl-1)-8 éthoxycarbonyl-3 fluoro-7 N-formyl N-méthylamino-1 oxo-4 dihydro-1,4 benzo[b]naphtyridine-1,8 dans 40 cm³ de potasse aqueuse N est chauffée, sous agitation, à une température voisine de 100°C, pendant 24 heures. Après refroidissement à environ 70°C, on élimine un léger insoluble par filtration. A cette même température, on ajoute au filtrat 40 cm³ d'acide méthanesulfonique N, essore le précipité formé et lave par 3 fois 20 cm³ d'eau à environ 70°C. Après recristallisation dans 100 cm³ de diméthylformamide, on obtient 1,05 g d'acide (amino-3 azétidinyl-1)-8 fluoro-7 méthylamino-1 oxo-4 dihydro-1,4 benzo[b]naphtyridine-1,8 carboxylique-3 sous forme d'un solide jaune se décomposant à 315-318°C.

L'(acétylamino-3 azétidinyl-1)-8 éthoxycarbonyl-3 fluoro-7 N-formyl N-méthylamino-1 oxo-4 dihydro-1,4 benzo[b]naphtyridine-1,8 a été préparé de la manière suivante:

A un mélange, à environ 30°C, de 1,13 g de chlorhydrate d'acétylamino-3 azétidine et de 0,85 g de carbonate de sodium dans 40 cm³ de diméthylsufoxyde, on ajoute 1,8 g de difluoro-7,8 éthoxycarbonyl-3 N- formyl N-méthylamino-1 oxo-4 dihydro-1,4 benzo[b]naphtyridine-1,8 et chauffe à environ 80°C, pendant 2 heures. Après refroidissement à environ 20°C, le mélange réactionnel est versé sur 100 cm³ d'eau à environ 5°C. Le précipité est essoré et lavé par 3 fois 50 cm³ d'eau. On obtient 2,05 g d'(acétylamino-3 azétidinyl-1)-8 éthoxycarbonyl-3 fluoro-7 N-formyl N-méthylamino-1 oxo-4 dihydro-1,4 benzo[b]naphty-ridine-1,8 sous forme d'un solide jaune se décomposant à 305°C.

La difluoro-7,8 éthoxycarbonyl-3 N-formyl N-méthylamino-1 oxo-4 dihydro-1,4 benzo[b]naphtyridine-1,8 a été préparée de la manière suivante:

A une solution sous agitation de 7,4 g de (chloro-2 difluoro-6,7 quinoléinecarbonyl-3)-2 diméthylamino-3 acrylate d'éthyle dans 30 cm³ de dichlorométhane, on ajoute en 10 minutes à environ 20°C, une solution de 1,63 g de N-formyl N-méthylhydrazine dans 30 cm³ de dichlorométhane. Après 16 heures à une température voisine de 20°C, le mélange réactionnel est concentré, sous pression réduite (20 kPa) à environ 40°C; L'extrait sec est repris par 100 cm³ d'éthanol, 3 cm³ de diaza-1,8-bicyclo [5.4.0.] undécène-7 (DBU) et chauffé à environ 75°C pendant 30 minutes. Après refroidissement à environ 20°C, le produit est essoré, lavé par 2 fois 50 cm³ d'éthanol et 2 fois 30 cm³ d'éther éthylique. On obtient 3,9 g de difluoro-7,8 éthoxycarbonyl-3 N-formyl N-méthylamino-1 oxo-4 dihydro-1,4 benzo[b]naphtyridine-1,8 sous forme d'un solide jaune fondant à 259-260°C qui est utilisé sans autre purification pour les étapes ultérieures.

Le (chloro-2 difluoro-6,7 quinoléinecarbonyl-3)-2 diméthylamino-3 acrylate d'éthyle a été préparé comme décrit dans le brevet US 4 970 213.

Le chlorhydrate d'acétylamino-3 azétidine a été préparé selon la méthode décrite par Dino Nisato et Coll. J. Heterocyclic Chem. 22, 961 (1985).

### EXEMPLE 11

Acide (trans-amino-3 méthyl-2 azétidinyl-1)-8 fluoro-7 méthyl-1 oxo-4 dihydro-1,4 benzo[b]naphtyridine-1,8 carboxylique-3.

Une solution de 18 g de diméthanesulfonate de trans-amino-3 méthyl-2 azétidine et de 12,7 g de triéthylamine dans 150 cm³ de diméthylsulfoxyde est chauffée sous agitation à environ 70°C pendant 20 minutes. A cette même température, on ajoute, par petites fractions, en 10 minutes, 14,5 g d'acide difluoro-7,8 méthyl-1 oxo-4 dihydro-1,4 benzo[b]naphtyridine-1,8 carboxylique-3 et chauffe à environ 90°C pendant 2 heures. Après refroidissement à environ 20°C, le mélange réactionnel est versé dans 400 cm³ d'eau à cette même température. L'insoluble est essoré, lavé par 2 fois 100 cm³ d'eau, 2 fois 100 cm³ d'éthanol et recristallisé dans 300 cm³ de diméthylformamide. On obtient 13,9 g d'acide (trans-amino-3 méthyl-2 azétidinyl-1)-8 fluoro-7 méthyl-1 oxo-4 dihydro-1,4 benzo[b]naphtyridine-1,8 carboxylique-3 sous forme d'un solide jaune se décomposant à 265-267°C.

La trans-amino-3 méthyl-2 azétidine a été préparée sous forme de diméthanesulfonate fondant à 170-175°C, selon la synthèse décrite dans la demande de brevet EP 406 112.

### EXEMPLE 12

Acide (cis-amino-3 méthyl-2 azétidinyl-1)-8 fluoro-7 méthyl-1 oxo-4 dihydro-1,4 benzo[b]naphtyridine-1,8 carboxylique-3.

On opère dans les conditions de l'exemple 11, mais à partir de 1,45 g d'acide difluoro-7,8 méthyl-1 oxo-4 dihydro-1,4 benzo[b]naphtyridine-1,8 carboxylique-3 et de 2,1 g de diméthanesulfonate de cis-amino-3 méthyl-2 azétidine. On obtient 1,32 g d'acide (cis-amino-3 méthyl-2 azétidinyl-1)-8 fluoro-7 méthyl-1 oxo-4 dihydro-1,4 benzo[b]naphtyridine-1,8 carboxylique-3 sous forme d'un solide jaune se décomposant à 312-315°C.

La cis-amino-3 méthyl-2 azétidine a été préparée sous forme de diméthanesulfonate fondant à 160-170°C selon la synthèse décrite dans la demande de brevet EP 406 112.

### EXEMPLE 13

Acide (trans-amino-3 méthyl-2 azétidinyl-1)-8 cyclopropyl-1 fluoro-7 oxo-4 dihydro-1,4 benzo[b]naphtyridine-1,8 carboxylique-3

Une suspension de 1,4 g de (trans-amino-3 méthyl-2 azétidinyl-1)-8 cyclopropyl-1 éthoxycarbonyl-3 fluoro-7 oxo-4 dihydro-1,4 benzo[b]naphtyridine-1,8 dans 15 cm³ d'eau et 6,8 cm³ de potasse aqueuse N est chauffée à environ 95°C pendant 3 heures. Après refroidissement à environ 60°C, on élimine un très léger insoluble par filtration; à la même température, on ajoute au filtrat 6,8 cm3 d'acide méthanesulfonique N. L'insoluble formé est essoré à environ 20°C, lavé par 3 fois 50 cm³ d'eau et 2 fois 25 cm³ d'éthanol. Après recristallisation dans 30 cm³ de diméthylformamide, on obtient. 1,03 g d'acide (trans-amino-3 méthyl-2 azétidinyl-1)-8 cyclopropyl-1 fluoro-7 oxo-4 dihydro-1,4 benzo[b]naphtyridine-1,8 carboxylique-3 sous forme d'un solide jaune se décomposant à 268-270°C.

La (trans-amino-3 méthyl-2 azétidinyl-1)-8 cyclopropyl-1 éthoxycarbonyl-3 fluoro-7 oxo-4 dihydro-1,4 benzo[b]naphtyridine-1,8 a été préparée de la manière suivante:

A une solution de diméthanesulfonate de trans-amino-3 méthyl-2 azétidine dans 20 cm³ de diméthylsufoxyde et 1,52 g de triéthylamine à environ 60°C, on ajoute en 10 minutes, par petites fractions 1,7 g de cyclopropyl-1 éthoxycarbonyl-3 difluoro-7,8 oxo-4 dihydro-1,4 benzo[b]naphtyridine-1,8. Le mélange réactionnel est chauffé à une température voisine de 80°C et maintenu, sous agitation, à cette même température pendant environ 4 heures. Après refroidissement à environ 20°C, le mélange réactionnel est versé dans 100 cm³ d'eau, extrait par 2 fois 100 cm³ de dichlorométhane. Les extraits organiques réunis sont lavés par 2 fois 50 cm³ d'eau, séchés sur sulfate de magnésium, concentrés à sec sous pression réduite (20 kPa) à environ 40°C. L'extrait sec est chromatographié sur 125 g de gel de silice (0,04-0,063 mm) en suspension dans un mélange de dichlorométhane à 20 % d'éthanol. Le produit cherché est élué par 300 cm³ du même mélange. On obtient 1,6 g de (trans-amino-3 méthyl-2 azétidinyl-1)-8 cyclopropyl-1 éthoxycarbonyl-3 fluoro-7 oxo-4 dihydro-1,4 benzo[b]naphtyridine-1,8 sous forme d'un solide jaune fondant à 170°C.

### EXEMPLE 14

Acide (amino-3 azétidinyl-1)-8 fluoro-7 oxo-4 tertiobutyl-1 dihydro-1,4 benze[b]naphtyridine-1,8 carboxylique-3.

Une suspension de 2,2 g de (trifluoroacétamido-3 azétidinyl-1)-8 éthoxycarbonyl-3 fluoro-7 oxo-4 tertiobutyl-1 dihydro-1,4 benzo[b]naphtyridine-1,6 dans 17 cm3 de potasse aqueuse N est chauffée à environ 95°C pendant 7 heures. A cette même température, on ajoute 17 cm3 d'acide méthanesulfonique N, essore à environ 90°C, lave par 2 fois 30 cm³ d'eau, 2 fois 30 cm3 d'éthanol et recristallise dans 50 cm³ de diméthylformamide. On obtient 1,4 g d'acide (amino-3 azétidinyl-1)-8 fluoro-7 oxo-4 tertiobutyl-1 dihydro-1,4 benzo[b]naphtyridine-1,8 carboxylique-3 sous forme d'un solide jaune se décomposant à 333-335°C.

L'éthoxycarbonyl-3 fluoro-7 (trifluoroacétamido-3 azétidinyl-1)-8 oxo-4 tertiobutyl-1 dihydro-1,4 benzo[b]naphtyridine-1,8 a été préparée dans les conditions décrites à l'exemple 2, mais à partir de de 1,8 g d'éthoxycarbonyl-3 difluoro-7,8 oxo-4 tertiobutyl-1 dihydro-1,4 benzo[b]naphtyridine-1,8, de 1,5 g de chlorhydrate de trifluoroacétamido-3 azétidine et de 0,79 g de carbonate de sodium. On obtient 2,25 g d'éthoxycarbonyl-3 fluoro-7 (trifluoroacétamido-3 azétidinyl-1)-8 oxo-4 tertiobutyl-1 dihydro-1,4 benzo[b]naphtyridine-1,8 sous forme d'un solide jaune fondant à 328-330°C.

L'éthoxycarbonyl-3 difluoro-7,8 oxo-4 tertiobutyl-1 dihydro-1,4 benzo[b]naphtyridine-1,8 a été préparée dans les conditions décrites à l'exemple 10, mais à partir de 4 g de (chloro-2 difluoro-6,7 quinoléinecarbonyl-3)-2 diméthylamino-3 acrylate d'éthyle et de 0,87 g de tertiobutylamine. On obtient 2,7 g d'éthoxycarbonl-3 difluoro-7,8 oxo-4 tertiobutyl-1 dihydro-1,4 benzo[b]naphtyridine-1-8 sous forme d'un solide jaune fondant à 206°C qui est utilisé sans autre purification pour les étapes ultérieures.

Le chlorhydrate de trifluoroacétamido-3 azétidine a été préparé comme décrit dans la demande de brevet EP 406 112.

### EXEMPLE 15

Acide (amino-3 propyl-3 azétidinyl-1)-8 fluoro-7 méthyl-1 oxo-4 dihydro-1,4 benzo[b]naphtyridine-1,8 carboxylique-3.

A une solution de 1,22 g d'éthylate de sodium dans 18 cm³ d'éthanol on ajoute 2,3 g de diméthanesulfonate d'amino-3 propyl-3 azétidine. Après addition de 30 cm³ de diméthylsulfoxyde, la solution est agitée pendant 10 minutes à environ 20°C. A cette même température, on ajoute 1,45 g d'acide difluoro-7,8 méthyl-1 oxo-4 dihydro-1,4 benzo[b]naphtyridine-1,8 carboxylique-3 et chauffe, sous agitation, à environ 90°C, pendant 5 heures. Après refroidissement à environ 20°C, le produit est essoré, lavé par 4 fois 20 cm³ d'eau et 2 fois 10 cm³ d'éthanol. Après recristallisation dans 30 cm³ de diméthylformamide, on obtient 1,52 g d'acide (amino-3 propyl-3 azétidinyl-1)-8 fluoro-7 méthyl-1 oxo-4 dihydro-1,4 benzo[b]naphtyridine-1,8 carboxylique-3 sous forme d'un solide jaune se décomposant à 304-305°C.

L'amino-3 propyl-3 azétidine sous forme de diméthanesulfonate, a été préparée dans des conditions voisines de celles décrites par D. Nisato et Coll., J. Heterocyclic, 22, 961 (1985).

Par hydrogénation de 7,3 g d'amino-3 benzhydryl-1 propyl-3 azétidine, sous une pression de 1 atmosphère, en présence de 5 g d'acide méthanesulfonique dans 75 cm³ de méthanol et de 1,5 g d'hydroxyde de palladium à 20 % sur charbon on obtient 6,5 g de diméthanesulfonate d'amino-3 propyl-3 azétidine. Le produit est repris dans l'éthanol et isolé sous forme d'un solide incolore fondant à 200°C.

L'amino-3 benzhydryl-1 propyl-3 azétidine a été préparée de la manière suivante:

A une solution à environ 5°C de 100 cm³ d'une solution d'ammoniac dans l'éthanol (préparée à partir de 15 g d'ammoniac dans 100 cm³ d'éthanol à 5°C, on ajoute 7,5 g de benzhydryl-1 méthanesulfonyloxy-3 propyl-3 azétidine, laisse la température remonter à environ 20°C et agite 16 heures à cette même température. Après concentration à sec sous pression réduite (20 kPa) à environ 40°C, le résidu est repris par 25 cm³ d'eau et 2,22 g d'acide méthanesulfonique. La phase aqueuse est lavée par 2 fois 25 cm³ d'éther éthylique, additionnée de 10 cm³ de soude aqueuse à 40 %, extraite par 3 fois 50 cm³ de dichlorométhane; les extraits organiques réunis sont lavés par 10 cm³ d'eau saturée de chlorure de sodium, séchés sur sulfate de magnésium et concentrés à sec dans les conditions précédentes. On obtient 5,2 g d'amino-3 benzhydryl-1 propyl-3 azétidine sous forme d'un produit huileux utilisé sans autre purification pour les étapes ultérieures.

La benzhydryl-1 méthanesulfonyloxy-3 propyl-3 azétidine a été préparée dans les conditions décrites pour la benzhydryl-1 méthanesulfonyloxy-3 méthyl-3 azétidine, dans la demande de brevet EP 406 112, mais à partir de 2,8 g de benzhydryl-1 hydroxy-3 propyl-3 azétidine. On obtient 2,4 g de benzhydryl-1 méthanesulfonyloxy-3 propyl-3 azétidine sous forme d'un solide incolore fondant à 70°C.

La benzhydryl-1 hydroxy-3 propyl-3 azétidine a été préparée dans les conditions suivantes:

A une solution d'iodure de propylmagnésium dans l'éther éthylique préparée dans les conditions habituelles à partir de 10,75 g d'iodopropane et de 1,55 g de magnésium dans 75 cm³ d'éther éthylique, on ajoute en 20 minutes, entre 0 et 5°C, une solution de 7,5 g de benzhydryl-1 oxo-3 azétidine en solution dans 50 cm³ d'éther éthylique. On laisse la température remonter à environ 20°C, et agite à cette même température pendant 2 heures. Après refroidissement à environ 0°C, on ajoute successivement en maintenant cette dernière température 50 cm³ d'eau et 50 cm3 d'une solution aqueuse à 10 % de chlorure d'ammonium. La phase aqueuse est extraite par 3 fois 50 cm³ d'éther éthylique, Les extraits organiques réunis sont séchés sur sulfate de magnésium, concentrés à sec sous pression réduite (20 kPa) à environ 30°C. L'extrait sec est chromatographié sur 100 g de gel de silice (0,04-0,063 mm) en suspension dans un mélange de cyclohexane à 20 % d'acétate d'éthyle. Le produit cherché est élué par 130 cm³ du même mélange de solvants. On obtient 8 g de benzhydryl-1 hydroxy-3 propyl-3 azétidine sous forme d'une huile jaune pâle qui est utilisée sans autre purification pour les étapes ultérieures.

La benzhydryl-1 oxo-3 azétidine a été préparée dans les conditions décrites par A. Morimoto et Coll. Chem. Pharm. Bull. 21(1) 228-231 (1973).

### EXEMPLE 16

Acide (amino-3 méthyl-3 azétidinyl-1)- 8 fluoro-7 méthyl-1 oxo-4 dihydro-1,4 benzo[b]naphtyridine-1,8 carboxylique-3.

A une solution de 1,02 g d'éthylate de sodium dans 30 cm3 d'éthanol à environ 5°C, on ajoute 2,09 g de diméthanesulfonate d'amino-3 méthyl-3 azétidine. On laisse la température remonter à environ 20°C, et agite 15 minutes à cette même température. Après élimination par filtration de sels minéraux, le filtrat est concentré à sec, sous pression réduite (20 kPa) à environ 30°C. Le résidu est mis en solution dans 20 cm³ de diméthylsulfoxyde est additionné à une température voisine de 20°C de 1,45 g d'acide difluoro-7,8 éthyl-1 oxo-4 dihydro-1,4 benzo[b]naphtyridine-1,8 carboxylique-3. La suspension obtenue est agitée 16 heures à cette même température, puis chauffée 1 heure à environ 60°C. Après refroidissement à environ 20°C, l'insoluble est essoré et lavé par 30 cm³ d'éthanol. Le produit isolé est recristallisé dans 90 cm³ de diméthylformamide, essoré et lavé par 20 cm³ d'éthanol à environ 75°C. On obtient 1,2 g d'acide (amino-3 méthyl-3 azétidinyl-1)-8 fluoro-7 méthyl-1 oxo-4 dihydro-1,4 benzo[b]naphtyridine-1,8 carboxylique-3 sous forme d'un solide jaune se décomposant à 345°C.

L'amino-3 méthyl-3 azétidine sous forme de diméthanesulfonate fondant à 204-206°C est préparée dans les conditions décrites dans la demande de brevet EP 406 112.

### EXEMPLE 17

L'acide (amino-3 méthyl-3 azétidinyl-1)-8 éthyl-1 fluoro-7 oxo-4 dihydro-1,4 benzo[b]naphtyridine-1,8 carboxylique-3 a été préparé dans des conditions décrites à l'exemple 15, mais à partir de 1,52 g d'acide difluoro-7,8 éthyl-1 oxo-4 dihydro-1,4 benzo[b]naphtyridine-1,8 carboxylique-3 et de 2,22 g de diméthanesulfonate d'amino-3 méthyl-3 azétidine pendant 72 heures à environ 20°C, puis 1 heure et demie à une température voisine de 90°C. L'insoluble du mélange réactionnel est essoré à environ 20°C, lavé par 2 fois 20 cm³ d'eau, recristallisé dans 70 cm³ de diméthylformamide, lavé par 40 cm³ d'éthanol à environ 75°C. On obtient 1,45 g d'acide (amino-3 méthyl-3 azétidinyl-1)-8 éthyl-1 fluoro-7 oxo-4 dihydro-1,4 benzo[b]naphtyridine-1,8 carboxylique-3 sous forme d'un solide jaune se décomposant à 334°C.

### EXEMPLE 18

L'acide (amino-3 méthyl-3 azétidinyl-1)-8 cyclopropyl-1 fluoro-7 oxo-4 dihydro-1,4 benzo[b]naphtyridine-1,8 carboxylique-3 a été préparé dans les conditions de l'exemple 16, mais à partir de 1,58 g d'acide cyclopropyl-1 difluoro-7,8 oxo-4 dihydro-1,4 benzo[b]naphtyridine-1,8 carboxylique-3 et de 2,22 g de diméthanesulfonate d'amino-3 méthyl-3 azétidine. On obtient 1,7 g d'acide (amino-3 méthyl-3 azétidinyl-1)-8 cyclopropyl-1 fluoro-7 oxo-4 dihydro-1,4 benzo[b]naphtyridine-1,8 carboxylique-3 sous forme d'un solide jaune se décomposant à 298°C.

L'acide cyclopropyl-1 difluoro-7,8 oxo-4 dihydro-1,4 benzo[b]naphtyridine-1,8 carboxylique-3 a été préparé dans les conditions suivantes:

Une suspension de 8,05 g de cyclopropyl-1 éthoxycarbonyl-3 difluoro-7,8 oxo-4 dihydro-1,4 benzo[b]naphtyridine-1,8 dans un mélange de 80 cm³ d'acide chlorhydrique en solution aqueuse à 17,5 % et de 80 cm³ d'acide acétique est chauffée sous agitation à une température voisine de 100°C pendant 2 heures. Après refroidissement à environ 20°C, le produit est essoré et lavé par 3 fois 100 cm³ d'eau. On obtient 6,25 g d'acide cyclopropyl-1 difluoro-7,8 oxo-4 dihydro-1,4 benzo[b]naphtyridine-1,8 carboxylique-3 sous forme d'un solide jaune pâle se décomposant à 283°C qui est utilisé sans autre purification pour les étapes ultérieures.

### EXEMPLE 19

L'acide (amino-3 diméthyl-2,2 azétidinyl-1)-8 fluoro-7 méthyl-1 oxo-4 dihydro-1,4 benzo[b]naphtyridine-1,8 carboxylique-3 (RS) a été préparé dans les conditions décrites à l'exemple 15, mais à partir de 1,45 g d'acide difluoro-7,8 méthyl-1 oxo-4 dihydro-1,4 benzo[b]naphtyridine-1,8 carboxylique-3 et de 2,33 g de diméthanesulfonate d'amino-3 diméthyl-2,2 azétidine. Le mélange réactionnel est chauffé, sous agitation, pendant 5 heures à environ 100°C. On obtient 0,6 g d'acide (amino-3 diméthyl-2,2 azétidinyl-1)-8 fluoro-7 méthyl-1 oxo-4 dihydro-1,4 ben-zo[b]naphtyridine-1,8 carboxylique-3 (RS) sous forme d'un solide jaune se décomposant à 285°C.

Le diméthanesulfonate d'amino-3 diméthyl-2,2 azétidine a été préparé sous forme d'un solide blanc fondant à 125-130°C à partir de l'amino-3 diméthyl-2,2 azétidine, par adaptation de la méthode décrite par Akira Morimoto Chem. Pharm. Bull., 21(1), 228 (1973).

### EXEMPLE 20

L'acide fluoro-7 méthyl-1 (méthyl-3 méthylamino-3 azétidinyl-1)-8 oxo-4 dihydro-1,4 benzo[b]naphtyridine-1,8 carboxylique-3 a été préparé dans les conditions de l'exemple 15, mais à partir de 1,45 g d'acide difluoro-7,8 méthyl-1 oxo-4 dihydro-1,4 benzo[b]naphtyridine-1,8 carboxylique-3 et de 2,34 g de diméthanesulfonate de méthyl-3 méthylamino-3 azétidine. Le mélange réactionnel est chauffé 1 heure et demie à environ 90°C. On obtient 1,12 g d'acide fluoro-7 méthyl-1 (méthyl-3 méthylamino-3 azétidinyl-1)-8 méthyl-1 oxo-4 dihydro-1,4 benzo[b]naphtyridine-1,8 carboxylique-3 sous forme d'un solide jaune se décomposant à environ 336°C.

La méthyl-3 méthylamino-3 azétidine sous forme de diméthanesulfonate (F=135°) a été préparée dans les conditions décrites dans la demande de brevet EP 406 112.

### EXEMPLE 21

L'acide cyclopropyl-1 fluoro-7 (méthyl-3 méthylamino-3 azétidlnyl-1)-8 oxo-4 dihydro-1,4 benzo[b]naphtyridine-1,8 carboxylique-3 a été préparé dans les conditions de l'exemple 15, mais à partir de 1,58 g d'acide difluoro-7,8 cyclopropyl-1 oxo-4 dihydro-1,4 benzo[b]naphtyridine-1,8 carboxylique-3 et de 2,34 g de diméthanesulfonate de méthyl-3 méthylamine-3 azétidine. On obtient 1,57 g d'acide cyclopropyl-1 fluoro-7 (méthyl-3 méthylamino-3 azétidinyl-1)-8 oxo-4 dihydro-1,4 benzo[b]naphtyridine-1,8 carboxylique-3 sous forme d'un solide jaune se décomposant à 330°C.

### EXEMPLE 22

L'acide éthyl-1 fluoro-7 (méthyl-3 méthylamino-3 azétidinyl-1)-8 oxo-4 dihydro-1,4 benzo[b]naphtyridine-1,8 carboxylique-3 a été préparé dans les conditions de l'exemple 15, mais à partir de 1,52 g d'acide difluoro-7,8 éthyl-1 oxo-4 dihydro-1,4 benzo[b]naphtyridine-1,8 carboxylique-3 et de 2,34 g de diméthanesulfonate de méthyl-3 méthylamino-3 azétidine. On obtient 1,18 g d'acide éthyl-1 fluoro-7 (méthyl-3 méthylamino-3 azétidinyl-1)-8 oxo-4 dihydro-1,4 benzo[b]naphtyridine-1,8 carboxylique-3 sous forme d'un solide jaune se décomposant à 338°C.

### EXEMPLE 23

L'acide (amino-3 méthyl-3 azétidinyl-1)-8 fluoro-7 méthoxy-1 oxo-4 dihydro-1,4 benzo[b]naphtyridine-1,8 carboxylique-3 a été préparé dans les conditions de l'exemple 15, mais à partir de 1,53 g d'acide difluoro-7,8 méthoxy-1 oxo-4 dihydro-1,4 benzo[b]naphtyridine-1,8 carboxylique-3 et de 2,22 g de diméthanesulfonate d'amino-3 méthyl-3 azétidine. On obtient 1,27 g d'acide (amino-3 méthyl-3 azétidinyl-1)-8 fluoro-7 méthoxy-1 oxo-4 dihydro-1,4 benzo[b]naphtyridine-1,8 carboxylique-3 sous forme d'un solide jaune se décomposant à 346°C.

L'acide difluoro-7,8 méthoxy-1 oxo-4 dihydro-1,4 benzo[b]naphtyridine-1,8 carboxylique-3 est préparé dans les conditions décrites dans le brevet US 4 970 213.

### EXEMPLE 24

L'acide cyclopropyl-1 fluoro-7 (méthyl-3 diméthylamino-3 azétidinyl-1)-8 oxo-4 dihydro-1,4 benzo[b]naphtyridine-1,8 carboxylique-3 a été préparé dans les conditions de l'exemple 15, mais à partir de 1,58 g d'acide cyclopropyl-1 difluoro-7,8 oxo-4 dihydro-1,4 benzo[b]naphtyridine-1,8 carboxylique-3 et de 2,45 g de diméthanesulfonate de méthyl-3 diméthylamino-3 azétidine. On obtient 1,55 g d'acide cyclopropyl-1 fluoro-7 (méthyl-3 diméthylamino-3 azétidinyl-1)-8 oxo-4 dihydro-1,4 benzo[b]naphtyridine-1,8 carboxylique-3 sous forme d'un solide jaune se décomposant à 268°C.

La méthyl-3 diméthylamino-3 azétidine sous forme de diméthanesulfonate fondant à 148°C est préparée dans les conditions décrites dans la demande de brevet EP 406 112.

### EXEMPLE 25

L'acide fluoro-7 méthyl-1 (méthyl-3 diméthylamino-3 azétidinyl-1)-8 oxo-4 dihydro-1,4 benzo[b]naphtyridine-1,8 carboxylique-3 a été préparé dans les conditions décrites à l'exemple 15, mais à partir de 1,45 g d'acide difluoro-7,8 méthyl-1 oxo-4 dihydro-1,4 benzo[b]naphtyridine-1,8 carboxylique-3 et de 2,45 g de diméthanesulfonate de méthyl-3 diméthylamino-3 azétidine. On obtient 1,56 g d'acide fluoro-7 méthyl-1 (méthyl-3 diméthylamino-3 azétidinyl-1)-8 oxo-4 dihydro-1,4 benzo[b]naphtyridine-1,8 carboxylique-3 sous forme d'un solide jaune se décomposant à 274°C.

### EXEMPLE 26

L'acide (amino-3 méthyl-3 azétidinyl-1)-8 difluoro-7,9 méthyl-1 oxo-4 dihydro-1,4 benzo[b]naptiyridine-1,8 carboxylique-3 a été préparé dans les conditions suivantes:

A une solution de 0,68 g d'éthylate de sodium dans 7 cm³ d'éthanol, on ajoute 1,33 g de diméthanesulfonate d'amino-3 méthyl-3 azétidine. Après addition de 20 cm³ de diméthylsulfoxyde, la solution est agitée pendant 10 minutes à environ 20°C. A cette même température, on ajoute 0,75 g d'acide trifluoro-7,8,9 méthyl-1 oxo-4 dihydro-1,4 benzo[b]naphtyridine-1,8 carboxylique-3 et agite pendant 72 heures à environ 20°C.

L'insoluble est essoré, lavé par 2 fois 20 cm³ d'eau et 2 fois 20 cm³ d'éthanol. Après recristallisation dans 70 cm³ de diméthylformamide, on obtient 0,45 g d'acide (amino-3 méthyl-3 azétidinyl-1)-8 difluoro-7,9 méthyl-1 oxo-4 dihydro-1,4 benzo[b]naphtyridine-1,8 carboxylique-3 sous forme d'un solide jaune se décomposant à 359°C.

L'acide trifluoro-7,8,9 méthyl-1 oxo-4 dihydro-1,4 benzo[b]naphtyridine-1,8 carboxylique-3 a été préparé de la manière suivante:

Une suspension de 0,88 g d'éthoxycarbonyl-3 trifluoro-7,8,9 méthyl-1 oxo-4 dihydro-1,4 benzo[b]naphtyridine-1,8 dans un mélange de 10 cm³ d'acide chlorhydrique en solution aqueuse à 17,5 % et de 10 cm³ d'acide acétique est chauffée sous agitation à une température voisine de 100°C pendant 3 heures. Après refroidissement à environ 20°C, le produit est essoré et lavé par 3 fois 10 cm³ d'eau. On obtient 0,75 g d'acide trifluoro-7,8,9 méthyl-1 oxo-4 dihydro-1,4 benzo[b]naphtyridine-1,8 carboxylique-3 sous forme d'un solide jaune pâle se décomposant à 352°C qui est utilisé sans autre purification pour les étapes ultérieures.

L'éthoxycarbonyl-3 trifluoro-7,8,9 méthyl-1 oxo-4 dihydro-1,4 benzo[b]naphtyridine-1,8 a été préparé dans les conditions décrites dans le brevet US 4 970 213.

### EXEMPLE 27

L'acide (cylopropylamino-3 méthyl-3 azétidinyl-1)-8 fluoro-7 méthyl-1 oxo-4 dihydro-1,4 benzo[b]naphtyridine-1,8 carboxylique-3 a été préparé dans les conditions de l'exemple 15, mais à partir de 1,45 g d'acide difluoro-7,8 méthyl-1 oxo-4 dihydro-1,4 benzo[b]naphtyridine-1,8 carboxylique-3 et de 2,5 g de diméthanesufonate de cyclopropylamino-3 méthyl-3 azétidine. On obtient 1,73 g d'acide (cylopropylamino-3 méthyl-3 azétidinyl-1)-8 fluoro-7 méthyl-1 oxo-4 dihydro-1,4 benzo[b]naphtyridine-1,8 carboxylique-3 solvaté par 0,7 % d'eau, sous forme d'un solide jaune se décomposant à 300°C.

Le diméthanesufonate de cyclopropylamino-3 méthyl-3 azétidine a été préparé de la manière suivante:

14,5 g de benzhydryl-1 cyclopropylamino-3 méthyl-3 azétidine dans 150 cm³ de méthanol sont hydrogénés sous pression atmosphérique, à une température voisine de 20°C, pendant 1 heure, en présence de 5,6 g d'hydroxyde de palladium à 20 % sur charbon. Le mélange réactionnel est additionné de 10 g d'acide méthanesulfonique. Après élimination du catalyseur par filtration, concentration à sec, sous pression réduite, (20 kPa) à environ 40°C, le résidu est lavé par 3 fois 150 cm³ d'éther éthylique. Le résidu final est repris par 100 cm³ de propanol-2, essoré, lavé par 2 fois 50 cm³ d'acétone. On obtient 9,1 g de diméthanesulfonate de cyclopropylamino-3 méthyl-3 azétidine sous forme d'un solide incolore fondant à 136°C.

La benzhydryl-1 cyclopropylamino-3 méthyl-3 azétidine a été préparée de la manière suivante:

Une suspension de 16,6 g de benzhydryl-1 méthanesulfonyloxy-3 méthyl-3 azétidine et de 28,84 g de cyclopropylamine dans 250 cm³ d'éthanol est agitée pendant 96 heures à environ 20°C. Le mélange réactionnel est concentré sous pression réduite (20 kPa) à environ 30°C. L'extrait sec est repris par 50 cm³ d'eau et 8,8 g d'acide méthanesulfonique ; la phase aqueuse est lavée par 3 fois 25 cm³ de dichlorométhane, additionnée de 12 cm³ de soude aqueuse à 35 % et extraite par 3 fois 50 cm³ d'acétate d'éthyle. Les phases organiques réunies sont lavées par 2 fois 50 cm³ d'eau, séchées sur sulfate de magnésium, concentrées à sec sous pression réduite (20 kPa) à environ 25°C. On obtient 14,6 g de benzhydryl-1 cyclopropylamino-3 méthyl-3 azétidine sous forme d'une huile jaune qui est utilisée sans autre purification pour les étapes ultérieures.

La benzhydryl-1 methanesulfonyloxy-3 méthyl-3 azétidine a été préparée selon les conditions décrites dans la demande de brevet EP 406 112.

### EXEMPLE 28

L'acide (éthylamino-3 méthyl-3 azétidinyl-1)-8 fluoro-7 méthyl-1 oxo-4 dihydro-1,4 benzo[b]naphtyridine-1,8 carboxylique-3 a été préparé dans les conditions de l'exemple 15, mais à partir de 1,45 g d'acide difluoro-7,8 méthyl-1 oxo-4 dihydro-1,4 benzo[b]naphtyridine-1,8 carboxylique-3 et de 2,24 g de diméthanesulfonate d'éthylamino-3 méthyl-3 azétidine. On obtient 1,66 g d'acide (éthylamino-3 méthyl-3 azétidinyl-1)-8 fluoro-7 méthyl-1 oxo-4 dihydro-1,4 benzo[b]naphtyridine-1,8 carboxylique-3 sous forme d'un solide jaune se décomposant à 312°C.

Le diméthanesulfonate d'éthylamino-3 méthyl-3 azétidine a été préparé dans les conditions décrites à l'exemple 27, mais à partir de 14 g de benzhydryl-1 éthylamino-3 méthyl-3 azétidine. On obtient 9,7 g de diméthanesulfonate d'éthylamino-3 méthyl-3 azétidine sous forme d'un solide incolore fondant à 140°C.

La benzhydryl-1 éthylamino-3 méthyl-3 azétidine a été préparée dans les conditions décrites à l'exemple 27, mais à partir de 16,6 g de benzhydryl-1 méthanesulfonyloxy-3 méthyl-3 azétidine et de 45 g d'éthylamine. On obtient 14 g de benzhydryl-1 éthylamino-3 méthyl-3 azétidine sous forme d'une huile jaune qui a été utilisée sans autre purification pour les étapes ultérieures.

### EXEMPLE 29

L'acide fluoro-7 (méthylamino-3 azétidinyl-1)-8 méthoxy-1 oxo-4 dihydro-1,4 benzo[b]naphtyridine-1,8 carboxylique-3 a été préparé dans les conditions de l'exemple 2, mais à partir de 2,6 g d'éthoxycarbonyl-3 fluoro-7 méthoxy-1 (méthylamino-3 azétidinyl-1)-8 oxo-4 dihydro-1,4 benzo[b]naphtyridine-1,8. On obtient 1,7 g d'acide fluoro-7 (méthylamino-3 azétidinyl-1)-8 méthoxy-1 oxo-4 dihydro-1,4 benzo[b]naphtyridine-1,8 carboxylique-3 sous forme d'un solide jaune se décomposant à 255-260°C.

L'éthoxycarbonyl-3 fluoro-7 méthoxy-1 (méthylamino-3 azétidinyl-1)-8 oxo-4 dihydro-1,4 benzo[b]naphtyridine-1,8 a été préparé dans les conditions suivantes:

A une solution de 2,36 g d'éthylate de sodium dans 20 cm³ d'éthanol, on ajoute 2,8 g de dichlorhydrate de méthylamino-3 azétidine. Après 15 minutes d'agitation à une température voisine de 20°C et élimination de sels minéraux par filtration, le filtrat est concentré à sec, sous pression réduite (20 kPa) à environ 30°C. Le résidu est mis en solution dans 40 cm³ de diméthylsulfoxyde et la solution additionnée de 2,7 g d'éthoxycarbonyl-3 difluoro-7,8 méthoxy-1 oxo-4 dihydro-1,4 benzo[b]naphtyridine-1,8. Le mélange réactionnel est chauffé 1 heure et demie à environ 60°C. On obtient 2,7 g d'éthoxycarbonyl-3 fluoro-7 (méthylamino-3 azétidinyl-1)-8 méthoxy-1 oxo-4 dihydro-1,4 benzo[b]naphtyridine-1,8 sous forme d'un solide jaune fondant à 234°C.

Le dichlorhydrate de méthylamino-3 azétidine a été préparé dans les conditions décrites à l'exemple 9, mais à partir de 32,2 de benzhydryl-1 méthylamino-3 azétidine. On obtient 19,1 g de dichlorhydrate de méthylamino-3 azétidine sous forme d'un solide incolore fondant à 138-140°C.

La benzhydryl-1 méthylamino-3 azétidine a été préparée dans les conditions de l'exemple 9, mais à partir de 50 g de benzhydryl-1 méthanesulfonyloxy-3 azétidine et de 48,9 g de méthylamine dans 250 cm³ de méthanol. On obtient 27 g de benzhydryl-1 méthylamino-3 azétidine sous forme d'un solide incolore fondant à 75°C.

### EXEMPLE 30

L'acide cyclopropyl-1 fluoro-7 (méthylamino-3 azétidinyl-1)-8 oxo-4 dihydro-1,4 benzo[b]naphtyridine-1,8 carboxylique-3 a été préparé dans les conditions de l'exemple 11, mais à partir de 1,5 g d'acide cyclopropyl-1 difluoro-7,8 oxo-4 dihydro-1,4 benzo[b]naphtyridine-1,8 carboxylique-3 et de 1,13 g de dichlorhydrate de méthylamino-3 azétidine. Le mélange réactionnel est agité pendant 1 heure à 40°C. On obtient 1 g d'acide cyclopropyl-1 fluoro-7 (méthylamino-3 azétidinyl-1)-8 oxo-4 dihydro-1,4 benzo[b]naphtyridine-1,8 carboxylique-3 sous forme d'un solide jaune se décomposant à 262°C.

### EXEMPLE 31

L'acide difluoro-7,9 méthyl-1 (méthylamino-3 azétidinyl-1)-8 oxo-4 dihydro-1,4 benzo[b]naphtyridine-1,8 carboxylique-3 a été préparé dans les conditions de l'exemple 30, mais à partir de 1,5 g d'acide trifluoro-7,8,9 méthyl-1 oxo-4 dihydro-1,4 benzo[b]naphtyridine-1,8 carboxylique-3 et de 1,2 g de dichlorhydrate de méthylamino-3 azétidine On obtient 1,54 g d'acide difluoro-7,9 méthyl-1 (méthylamino-3 azétidinyl-1)-8 oxo-4 dihydro-1,4 benzo[b]naphtyridine-1,8 carboxylique-3 sous forme d'un solide jaune se décomposant à 302°C.

### EXEMPLE 32

L'acide (amino-3 éthyl-3 azétidinyl-1)-8 fluoro-7 méthyl-1 oxo-4 dihydro-1,4 benzo[b]naphtyridine-1,8 carboxylique-3 a été préparé dans les conditions de l'exemple 15, mais à partir de 1,66 g d'acide difluoro-7,8 méthyl-1 oxo-4 dihydro-1,4 benzo[b]naphtyridine-1,8 carboxylique-3 et de 3,1 g de diméthanesulfonate d'amino-3 éthyl-3 azétidine. On obtient 1,46 g d'acide (amino-3 éthyl-3 azétidinyl-1)-8 fluoro-7 méthyl-1 oxo-4 dihydro-1,4 benzo[b]naphtyridine-1,8 carboxylique-3 sous forme d'un solide jaune se décomposant à 294°C.

Le diméthanesulfonate d'amino-3 éthyl-3 azétidine a été préparé selon les conditions décrites à l'exemple 15 pour le diméthanesulfonate d'amino-3 propyl-3 azétidine : 5,7 g de diméthanesulfonate d'amino-3 éthyl-3 azétidine ont été obtenus sous forme d'un solide incolore fondant à 184°C, par hydrogénation de 6 g d'amino-3 benzhydryl-1 éthyl-3 azétidine.

13,6 g d'amino-3 benzhydryl-1 éthyl-3 azétidine ont été obtenus sous forme d'une huile jaune à partir de 24,6 g de benzhydryl-1 éthyl-3 méthanesulfonyloxy-3 azétidine en solution dans 174 cm³ d'une solution d'ammoniac à 15 % dans l'éthanol. Le produit isolé a été utilisé sans autre purification pour les étapes ultérieures.

24,6 g de benzhydryl-1 éthyl-3 méthanesulfonyloxy-3 azétidine ont été obtenus sous forme d'un solide jaune fondant à 68°C, à partir de 35,5 g de benzhydryl-1 hydroxy-3 éthyl-3 azétidine.

35,5 g de benzhydryl-1 hydroxy-3 éthyl-3 azétidine ont été obtenus sous forme d'une huile jaune à partir de 44 g de benzhydryl-1 oxo-3 azétidine et de 80 cm³ de bromure d'éthyle magnésium à 399 g par litre dans l'éther.

### EXEMPLE 33

Le méthanesulfonate de l'acide (amino-3 éthyl-3 azétidinyl-1)-8 cyclopropyl-1 fluoro-7 oxo-4 dihydro-1,4 benzo[b]naphtyridine-1,8 carboxylique-3 a été préparé dans les conditions de l'exemple 15, mais à partir de 1,8 g d'acide difluoro-7,8 cylopropyl-1 oxo-4 dihydro-1,4 benzo[b]naphtyridine-1,8 carboxylique-3 et de 2,94 g de diméthanesulfonate d'amino-3 éthyl-3 azétidine. Après recristallisation dans le diméthylformamide, le produit obtenu mis en suspension dans 20 cm³ d'éthanol est additionné de 3,4 cm³ d'une solution à 9,6 g % d'acide méthanesulfonique dans l'éthanol. La suspension est agitée 5 minutes à environ 80°C, refroidie à une température voisine de 20°C, essorée et lavée par 3 fois 10 cm³ d'éthanol. On obtient 0,55 g de méthanesulfonate de l'acide (amino-3 éthyl-3 azétidinyl-1)-8 cyclopropyl-1 fluoro-7 oxo-4 dihydro-1,4 benzo[b]naphtyridine-1,8 carboxylique-3 sous forme d'un solide jaune se décomposant à 248°C.

### EXEMPLE 34

L'acide fluoro-7 méthyl-1 [(pyrrolidinyl-1)-3 azétidinyl-1]-8 oxo-4 dihydro-1,4 benzo[b]naphtyridine-1,8 carboxylique-3 a été préparé dans les conditions de l'exemple 2, mais à partir de 1,2 g d'éthoxycarbonyl-3 fluoro-7 méthyl-1 [(pyrrolidinyl-1)-3 azétidinyl-1]-8 oxo-4 dihydro-1,4 benzo[b]naphtyridine-1,8. On obtient 0,7 g d'acide fluoro-7 méthyl-1 [(pyrrolidinyl-1)-3 azétidinyl-1]-8 oxo-4 dihydro-1,4 benzo[b]naphtyridine-1,8 carboxylique-3 sous forme d'un solide jaune se décomposant à 302°C.

L'éthoxycarbonyl-3 fluoro-7 méthyl-1 [(pyrrolidinyl-1)-3 azétidinyl-1]-8 oxo-4 dihydro-1,4 benzo[b]naphtyridine-1,8 a été préparé dans les conditions de l'exemple 2, mais à partir de 1,6 g d'éthoxycarbonyl-3 difluoro-7,6 méthyl-1 oxo-4 dihydro-1,4 benzo[b]naphtyridine-1,8 et de 1,5 g de dichlorhydrate de (pyrrolidinyl-1)-3 azétidine. Le mélange réactionnel est agité pendant 24 heures à environ 95°C. On obtient 1,3 g d'éthoxycarbonyl-3 fluoro-7 méthyl-1 [(pyrrolidinyl-1)-3 azétidinyl-1]-8 oxo-4 dihydro-1,4 benzo[b]naphtyridine-1,8 sous forme d'un solide jaune fondant à 246°C.

La (pyrrolidinyl-1)-3 azétidine a été préparée sous forme de dichlorhydrate fondant à 195°C, dans les conditions décrites dans la demande de brevet japonais 74 109 369.

### EXEMPLE 35

L'acide (cyclopropylamino-3 azétidinyl-1)-8 fluoro-7 méthyl-1 oxo-4 dihydro-1,4 benzo[b]naphtyridine-1,8 carboxylique-3 a été préparé dans les conditions de l'exemple 2, mais à partir de 1,3 g de (cyclopropylamino-3 azétidinyl-1)-8 éthoxycarbonyl-3 fluoro-7 méthyl-1 oxo-4 dihydro-1,4 benzo[b]naphtyridine-1,8. On obtient 0,8 g d'acide (cyclopropylamino-3 azétidinyl-1)-8 fluoro-7 méthyl-1 oxo-4 dihydro-1,4 benzo[b]naphtyridine-1,8 carboxylique-3 sous forme d'un solide jaune se décomposant à 272°C.

La (cyclopropylamino-3 azétidinyl-1)-8 éthoxycarbonyl-3 fluoro-7 méthyl-1 oxo-4 dihydro-1,4 benzo[b]naphtyridine-1,8 a été préparée dans les conditions de l'exemple 15, mais à partir de 1,45 g d'éthoxy-carbonyl-3 difluoro-7,8 méthyl-1 oxo-4 dihydro-1,4 benzo[b]naphtyridine-1,8 et de 1,7 g de dichlorhydrate de cyclopropylamino-3 azétidine. On obtient 1,35 g de (cyclopropylamino-3 azétidinyl-1)-8 éthoxycarbonyl-3 fluoro-7 méthyl-1 oxo-4 dihydro-1,4 benzo[b]naphtyridine-1,8 sous forme d'un solide jaune fondant à 221°C puis 245°C.

Le dichlorhydrate de cyclopropylamino-3 azétidine a été préparé dans les conditions décrites à l'exemple 9 pour le dichlorhydrate d'éthylamino-3 azétidine, mais à partir de 5,4 g de dichlorhydrate de benzhydryl-1 cyclopropylamino-3 azétidine. On obtient 2,25 g de dichlorhydrate de cyclopropylamino-3 azétidine sous forme d'un solide incolore fondant à 140°C.

Le dichlorhydrate de benzhydryl-1 cyclopropylamino-3 azétidine a été préparé dans les conditions décrites à l'exemple 9 pour le dichlorhydrate de benzhydryl-1 éthylamino-3 azétidine, mais à partir de 15 g de benzhydryl-1 méthanesulfonyloxy-3 azétidine et de 8,2 g de cyclopropylamine. On obtient 5,4 g de dichlorhydrate de benzhydryl-1 cyclopropylamino-3 azétidine sous forme d'un solide incolore fondant à 182°C.

### EXEMPLE 36

En opérant comme décrit dans les exemples précédents, on prépare les produits suivants :
- acide fluoro-7 méthyl-1 (méthylamino-3 azétidinyl-1)-8 oxo-4 dihydro-1,4 benzo[b]naphtyridine-1,8 carboxylique-3 ;
- acide [(amino-2 éthyl)amino-3 azétidinyl-1]-8 fluoro-7 méthyl-1 oxo-4 dihydro-1,4 benzo[b]naphtyridine-1,8 carboxylique-3 ;
- acide fluoro-7 [(hydroxy-2 éthyl)amino-3 azétidinyl-1]-8 méthyl-1 oxo-4 dihydro-1,4 benzo[b]naphtyridine-1,8 carboxylique-3 ;
- acide fluoro-7 méthyl-1 oxo-4 [(pipérazinyl-1)-3 azétidinyl-1]-8 dihydro-1,4 benzo[b]naphtyridine-1,8 carboxylique-3 ;
- acide {[(amino-2 éthyl)-4 phényl] amino-3 azétidinyl-1}-8 fluoro-7 méthyl-1 oxo-4 dihydro-1,4 benzo[b]naphtyridine-1,8 carboxylique-3 ;
- acide (amino-3 phényl-3 azétidinyl-1)-8 fluoro-7 méthyl-1 oxo-4 dihydro-1,4 benzo[b]naphtyridine-1,8 carboxylique-3 ;
- acide (amino-3 diméthylamino-2 azétidinyl-1]-8 fluoro-7 méthyl-1 oxo-4 dihydro-1,4 benzo[b]naphtyridine-1,8 carboxylique-3 ;
- acide éthyl-1 fluoro-7 (méthylamino-3 azétidinyl-1)-8 oxo-4 dihydro-1,4 benzo[b]naphtyridine-1,8 carboxylique-3 ;
- acide (éthylamino-3 azétidinyl-1)-8 éthyl-1 fluoro-7 oxo-4 dihydro-1,4 benzo[b]naphtyridine-1,8 carboxylique-3 ;
- acide [(amino-2 éthyl)amino-3 azétidinyl-1]-8 éthyl-1 fluoro-7 oxo-4 dihydro-1,4 benzo[b]naphtyridine-1,8 carboxylique-3 ;
- acide éthyl-1 fluoro-7 [(hydroxy-2 éthyl)amino-3 azétidinyl-1]-8 oxo-4 dihydro-1,4 benzo[b]naphtyridine-1,8 carboxylique-3 ;
- acide (cyclopropylamino-3 azétidinyl-1)-8 éthyl-1 fluoro-7 oxo-4 dihydro-1,4 benzo[b]naphtyridine-1,8 carboxylique-3 ;
- acide éthyl-1 fluoro-7 oxo-4 [(pipérazinyl-1)-3 azétidinyl-1]-8 dihydro-1,4 benzo[b]naphtyridine-1,8 carboxylique-3 ;
- acide {[(amino-2 éthyl)-4 phényl] amino-3 azétidinyl-1}-8 éthyl-1 fluoro-7 oxo-4 dihydro-1,4 benzo[b]naphtyridine-1,8 carboxylique-3 ;
- acide (amino-3 phényl-3 azétidinyl-1)-8 éthyl-1 fluoro-7 oxo-4 dihydro-1,4 benzo[b]naphtyridine-1,8 carboxylique-3 ;
- acide (amino-3 diméthylamino-2 azétidinyl-1)-8 éthyl-1 fluoro-7 oxo-4 dihydro-1,4 benzo[b]naphtyridine-1,8 carboxylique-3 ;
- acide cyclopropyl-1 (éthylamino-3 azétidinyl-1)-8 fluoro-7 oxo-4 dihydro-1,4 benzo[b]naphtyridine-1,8 carboxylique-3 ;
- acide [(amino-2 éthyl)amino-3 azétidinyl-1]-8 cyclopropyl-1 fluoro-7 oxo-4 dihydro-1,4 benzo[b]naphtyridine-1,8 carboxylique-3 ;
- acide cyclopropyl-1 fluoro-7 [(hydroxy-2 éthyl)amino-3 azétidinyl-1]-8 oxo-4 dihydro-1,4 benzo[b]naphtyridine-1,8 carboxylique-3 ;
- acide cyclopropyl-1 (cyclopropylamino-3 azétidinyl-1)-8 fluoro-7 oxo-4 dihydro-1,4 benzo[b]naphtyridine-1,8 carboxylique-3 ;
- acide cyclopropyl-1 fluoro-7 oxo-4 [(pipérazinyl-1)-3 azétidinyl-1]-8 dihydro-1,4 benzo[b]naphtyridine-1,8 carboxylique-3 ;
- acide {[(amino-2 éthyl)-4 phényl] amino-3 azétidinyl-1}-8 cyclopropyl-1 fluoro-7 oxo-4 dihydro-1,4 benzo[b]naphtyridine-1,8 carboxylique-3 ;
- acide (amino-3 phényl-3 azétidinyl-1)-8 cyclopropyl-1 fluoro-7 oxo-4 dihydro-1,4 benzo[b]naphtyridine-1,8 carboxylique-3 ;
- acide (amino-3 diméthylamino-2 azétidinyl-1)-8 cyclopropyl-1 fluoro-7 oxo-4 dihydro-1,4 benzo[b]naphtyridine-1,8 carboxylique-3 ;
- acide fluoro-7 méthylamino-1 (méthylamino-3 azétidinyl-1)-8 oxo-4 dihydro-1,4 benzo[b]naphtyridine-1,8 carboxylique-3 ;
- acide (éthylamino-3 azétidinyl-1)-8 fluoro-7 méthylamino-1 oxo-4 dihydro-1,4 benzo[b]naphtyridine-1,8 carboxylique-3 ;
- acide [(amino-2 éthyl)amino-3 azétidinyl-1]-8 fluoro-7 méthylamino-1 oxo-4 dihydro-1,4 benzo[b]naphtyridine-1,8 carboxylique-3 ;
- acide fluoro-7 [(hydroxy-2 éthyl)amino-3 azétidinyl-1]-8 méthylamino-1 oxo-4 dihydro-1,4 benzo[b]naphtyridine-1,8 carboxylique-3 ;
- acide (cyclopropylamino-3 azétidinyl-1)-8 fluoro-7 méthylamino-1 oxo-4 dihydro-1,4 benzo[b]naphtyridine-1,8 carboxylique-3 ;
- acide fluoro-7 mécthylamino-1 7 oxo-4 [(pipérazinyl-1)-3 azétidinyl-1]-8 dihydro-1,4 benzo[b]naphtyridine-1,8 carboxylique-3 ;
- acide {[(amino-2 éthyl)-4 phényl] amino-3 azétidinyl-1}-8 fluoro-7 méthylamino-1 oxo-4 dihydro-1,4 benzo[b]naphtyridine-1,8 carboxylique-3 ;
- acide (amino-3 phényl-3 azétidinyl-1)-8 fluoro-7 méthylamino-1 oxo-4 dihydro-1,4 benzo[b]naphtyridine-1,8 carboxylique-3 ;
- acide (amino-3 méthyl-3 azétidinyl-1)-8 fluoro-7 méthylamino-1 oxo-4 dihydro-1,4 benzo[b]naphtyridine-1,8 carboxylique-3 ;
- acide (amino-3 diméthylamino-2 azétidinyl-1)-8 fluoro-7 méthylamino-1 oxo-4 dihydro-1,4 benzo[b]naphtyridine-1,8 carboxylique-3 ;
- acide fluoro-7 (fluoro-2 éthyl)-1 (méthylamino-3 azétidinyl-1)-8 oxo-4 dihydro-1,4 benzo[b]naphtyridine-1,8 carboxylique-3 ;
- acide (éthylamino-3 azétidinyl-1)-8 fluoro-7 (fluoro-2 éthyl)-1 oxo-4 dihydro-1,4 benzo[b]naphtyridine-1,8 carboxylique-3 ;
- acide [(amino-2 éthyl)amino-3 azétidinyl-1]-8 fluoro-7 (fluoro-2 éthyl)-1 oxo-4 dihydro-1,4 benzo[b]naphtyridine-1,8 carboxylique-3 ;
- acide fluoro-7 (fluoro-2 éthyl)-1 [(hydroxy-2 éthyl)amino-3 azétidinyl-1]-8 oxo-4 dihydro-1,4 benzo[b]naphtyridine-1,8 carboxylique-3 ;
- acide (cyclopropylamino-3 azétidinyl-1)-8 fluoro-7 (fluoro-2 éthyl)-1 oxo-4 dihydro-1,4 benzo[b]naphtyridine-1,8 carboxylique-3 ;
- acide fluoro-7 (fluoro-2 éthyl)-1 oxo-4 [(pipérazinyl-1)-3 azétidinyl-1]-8 dihydro-1,4 benzo[b]naphtyridine-1,8 carboxylique-3 ;
- acide {[(amino-2 éthyl)-4 phényl] amino-3 azétidinyl-1}-8 fluoro-7 (fluoro-2 éthyl)-1 oxo-4 dihydro-1,4 benzo[b]naphtyridine-1,8 carboxylique-3 ;
- acide (amino-3 phényl-3 azétidinyl-1)-8 fluoro-7 (fluoro-2 éthyl)-1 oxo-4 dihydro-1,4 benzo[b]naphtyridine-1,8 carboxylique-3 ;
- acide (amino-3 méthyl-3 azétidinyl-1)-8 fluoro-7 (fluoro-2 éthyl)-1 oxo-4 dihydro-1,4 benzo[b]naphtyridine-1,8 carboxylique-3 ;
- acide (amino-3 diméthylamino-2 azétidinyl-1)-8 fluoro-7 (fluoro-2 éthyl)-1 oxo-4 dihydro-1,4 benzo[b]naphtyridine-1,8 carboxylique-3 ;
- acide fluoro-7 (méthylamino-3 azétidinyl-1)-8 oxo-4 t.butyl-1 dihydro-1,4 benzo[b]naphtyridine-1,8 carboxylique-3 ;
- acide (éthylamino-3 azétidinyl-1)-8 fluoro-7 oxo-4 t.butyl-1 dihydro-1,4 benzo[b]naphtyridine-1,8 carboxylique-3 ;
- acide [(amino-2 éthyl)amino-3 azétidinyl-1]-8 fluoro-7 oxo-4 t.butyl-1 dihydro-1,4 benzo[b]naphtyridine-1,8 carboxylique-3 ;
- acide fluoro-7 [(hydroxy-2 éthyl)amino-3 azétidinyl-1]-8 oxo-4 t.butyl-1 dihydro-1,4 benzo[b]naphtyridine-1,8 carboxylique-3 ;
- acide (cyclopropylamino-3 azétidinyl-1)-8 fluoro-7 oxo-4 t.butyl-1 dihydro-1,4 benzo[b]naphtyridine-1,8 carboxylique-3 ;
- acide fluoro-7 oxo-4 [(pipérazinyl-1)-3 azétidinyl-1]-8 t.butyl-1 dihydro-1,4 benzo[b]naphtyridine-1,8 carboxylique-3 ;
- acide {[(amino-2 éthyl)-4 phényl] amino-3 azétidinyl-1}-8 fluoro-7 oxo-4 t.butyl-1 dihydro-1,4 benzo[b]naphtyridine-1,8 carboxylique-3 ;
- acide (amino-3 phényl-3 azétidinyl-1)-8 fluoro-7 oxo-4 t.butyl-1 dihydro-1,4 benzo[b]naphtyridine-1,8 carboxylique-3 ;
- acide (amino-3 méthyl-3 azétidinyl-1)-8 fluoro-7 oxo-4 t.butyl-1 dihydro-1,4 benzo[b]naphtyridine-1,8 carboxylique-3 ;
- acide (amino-3 diméthylamino-2 azétidinyl-1)-8 fluoro-7 oxo-4 t.butyl-1 dihydro-1,4 benzo[b]naphtyridine-1,8 carboxylique-3 ;
- acide difluoro-7,9 (éthylamino-3 azétidinyl-1)-8 méthyl-1 oxo-4 dihydro-1,4 benzo[b]naphtyridine-1,8 carboxylique-3 ;
- acide [(amino-2 éthyl)amino-3 azétidinyl-1]-8 difluoro-7,9 méthyl-1 oxo-4 dihydro-1,4 benzo[b]naphtyridine-1,8 carboxylique-3 ;
- acide difluoro-7,9 [(hydroxy-2 éthyl)amino-3 azétidinyl-1]-8 méthyl-1 oxo-4 dihydro-1,4 benzo[b]naphtyridine-1,8 carboxylique-3 ;
- acide (cyclopropylamino-3 azétidinyl-1)-8 difluoro-7,9 méthyl-1 oxo-4 dihydro-1,4 benzo[b]naphtyridine-1,8 carboxylique-3 ;
- acide difluoro-7,9 méthyl-1 oxo-4 [(pipérazinyl-1)-3 azétidinyl-1]-8 dihydro-1,4 benzo[b]naphtyridine-1,8 carboxylique-3 ;
- acide {[(amino-2 éthyl)-4 phényl] amino-3 azétidinyl-1}-8 difluoro-7,9 méthyl-1 oxo-4 dihydro-1,4 4 benzo[b]naphtyridine-1,8 carboxylique-3 ;
- acide (amino-3 phényl-3 azétidinyl-1)-8 difluoro-7,9 méthyl-1 oxo-4 dihydro-1,4 benzo[b]naphtyridine-1,8 carboxylique-3 ;
- acide (amino-3 diméthylamino-2 azétidinyl-1)-8 difluoro-7,9 méthyl-1 oxo-4 dihydro-1,4 benzo[b]naphtyridine-1,8 carboxylique-3 ;
- acide difluoro-7,9 éthyl-1 (méthylamino-3 azétidinyl-1)-8 oxo-4 dihydro-1,4 benzo[b]naphtyridine-1,8 carboxylique-3 ;
- acide (éthylamino-3 azétidinyl-1)-8 difluoro-7,9 éthyl-1 oxo-4 dihydro-1,4 benzo[b]naphtyridine-1,8 carboxylique-3 ;
- acide [(amino-2 éthyl)amino-3 azétidinyl-1]-8 difluoro-7,9 éthyl-1 oxo-4 dihydro-1,4 benzo[b]naphtyridine-1,8 carboxylique-3 ;
- acide difluoro-7,9 éthyl-1 [(hydroxy-2 éthyl)amino-3 azéti-dinyl-1]-8 oxo-4 dihydro-1,4 benzo[b]naphtyridine-1,8 carboxylique-3 ;
- acide (cyclopropylamino-3 azétidinyl-1)-8 difluoro-7,9 éthyl-1 oxo-4 dihydro-1,4 benzo[b]naphtyridine-1,8 carboxylique-3 ;
- acide difluoro-7,9 éthyl-1 oxo-4 [(pipérazinyl-1)-3 azétidinyl-1]-8 dihydro-1,4 benzo[b]naphtyridine-1,8 carboxylique-3 ;
- acide {[(amino-2 éthyl)-4 phényl] amino-3 azétidinyl-1}-8 difluoro-7,9 éthyl-1 oxo-4 dihydro-1,4 benzo[b]naphtyridine-1,8 carboxylique-3 ;
- acide (amino-3 phényl-3 azétidinyl-1)-8 difluoro-7,9 éthyl-1 oxo-4 dihydro-1,4 benzo[b]naphtyridine-1,8 carboxylique-3 ;
- acide (amino-3 methyl-3 azétidinyl-1)-8 difluoro-7,9 éthyl-1 oxo-4 dihydro-1,4 benzo[b]naphtyridine-1,8 carboxylique-3 ;
- acide (amino-3 diméthylamino-2 azétidinyl-1)-8 difluoro-7,9 éthyl-1 oxo-4 hydro-1,4 benzo[b]naphtyridine-1,8 carboxylique-3 ;
- acide cyclopropyl-1 difluoro-7,9 (méthylamino-3 azétidinyl-1)-8 oxo-4 dihydro-1,4 benzo[b]naphtyridine-1,8 carboxylique-3 ;
- acide cyclopropyl-1 (éthylamino-3 azétidinyl-1)-8 difluoro-7,9 oxo-4 dihydro-1,4 benzo[b]naphtyridine-1,8 carboxylique-3 ;
- acide [(amino-2 éthyl)amino-3 azétidinyl-1]-8 cyclopropyl-1 difluoro-7,8 oxo-4 dihydro-1,4 benzo[b]naphtyridine-1,8 carboxylique-3 ;
- acide cyclopropyl-1 difluoro-7,9 [(hydroxy-2 éthyl)amino-3 azétidinyl-1]-8 oxo-4 dihydro-1,4 benzo[b]naphtyridine-1,8 carboxylique-3 ;
- acide cyclopropyl-1 (cyclopropylamino-3 azétidinyl-1)-8 difluoro-7,8 oxo-4 dihydro-1,4 benzo[b]naphtyridine-1,8 carboxylique-3 ;
- acide cyclopropyl-1 difluoro-7,9 oxo-4 [(pipérazinyl-1)-3 azétidinyl-1]-8 dihydro-1,4 benzo[b]naphtyridine-1,8 carboxylique-3 ;
- acide {[(amino-2 éthyl)-4 phényl] amino-3 azétidinyl-1}-8 cyclopropyl-1 difluoro-7,9 oxo-4 dihydro-1,4 benzo[b]naphtyridine-1,8 carboxylique-3 ;
- acide (amino-3 phényl-3 azétidinyl-1)-8 cyclopropyl-1 difluoro-7,9 oxo-4 dihydro-1,4 benzo[b]naphtyridine-1,8 carboxylique-3 ;
- acide (amino-3 méthyl-3 azétidinyl-1)-8 cyclopropyl-1 difluoro-7,9 oxo-4 dihydro-1,4 benzo[b]naphtyridine-1,8 carboxylique-3 ;
- acide (amino-3 diméthylamino-2 azétidinyl-)-8 cyclopropyl-1 difluoro-7,8 oxo-4 dihydro-1,4 benzo[b]naphtyridine-1,8 carboxylique-3 ;
- acide difluoro-7,9 méthylamino-1 (méthylamino-3 azétidinyl-1)-8 oxo-4 dihydro-1,4 benzo[b]naphtyridine-1,8 carboxylique-3 ;
- acide (éthylamino-3 azétidiny-1)-8 difluoro-7,9 méthylamino-1 oxo-4 dihydro-1,4 benzo[b]naphtyridine-1,8 carboxylique-3 ;
- acide [(amino- éthyl)amino-3 azétidinyl-1]-8 difluoro-7,9 méthylamino-1 oxo-4 dihydro-1,4 benzo[b]naphtyridine-1,8 carboxylique-3 ;
- acide difluoro-7,9 [(hydroxy-2 éthyl)amino-3 azétidinyl-1]-8 méthylamino-1 oxo-4 dihydro-1,4 benzo[b]naphtyridine-1,8 carboxylique-3 ;
- acide (cyclopropylamino-3 azétidinyl-1)-8 difluoro-7,9 méthylamino-1 oxo-4 dihydro-1,4 benzo[b]naphtyridine-1,8 carboxylique-3 ;
- acide difluoro-7,9 méthylamino-1 oxo-4 [(pipérazinyl-1)-3 azétidinyl-1]-8 dihydro-1,4 benzo[b]naphtyridine-1,8 carboxylique-3 ;
- acide {[(amino-2 éthyl)-4 phényl] amino-3 azétidinyl-1}-8 difluoro-7,9 méthylamino-1 oxo-4 dihydro-1,4 benzo[b]naphtyridine-1,8 carboxylique-3 ;
- acide (amino-3 phényl-3 azétidinyl-1)-8 difluoro-7,9 méthylamino-1 oxo-4 dihydro-1,4 benzo[b]naphtyridine-1,8 carboxylique-3 ;
- acide (amino-3 méthyl-3 azétidinyl-1)-8 difluoro-7,9 méthylamino-1 oxo-4 dihydro-1,4 benzo[b]naphtyridine-1,8 carboxylique-3 ;
- acide (amino-3 diméthylamino-2 azétidinyl-1)-8 difluoro-7,9 méthylamino-1 oxo-4 dihydro-1,4 benzo[b]naphtyridine-1,8 carboxylique-3 ;
- acide difluoro-7,9 (fluoro-2 éthyl)-1 (méthylamino-3 azétidinyl-1)-8 oxo-4 dihydro-1,4 benzo[b]naphtyridine-1,8 carboxylique-3 ;
- acide difluoro-7,9 (éthylamino-3 azétidinyl-1)-8 (fluoro-2 éthyl)-1 oxo-4 dihydro-1,4 benzo[b]naphtyridine-1,8 carboxylique-3 ;
- acide [(amino-2 éthyl)amino-3 azétidinyl-1]-8 difluoro-7,9 (fluoro-2 éthyl)-1 oxo-4 dihydro-1,4 benzo[b]naphtyridine-1,8 carboxylique-3 ;
- acide difluoro-7,9 (fluoro-2 éthyl)-1 [(hydroxy-2 éthyl)amino-3 azétidinyl-1]-8 oxo-4 dihydro-1,4 benzo[b]naphtyridine-1,8 carboxylique-3 ;
- acide (cyclopropylamino-3 azétidinyl-1)-8 difluoro-7,9 (fluoro-2 éthyl)-1 oxo-4 dihydro-1,4 benzo[b]naphtyridine-1,8 carboxylique-3 ;
- acide difluoro-7,9 (fluoro-2 éthyl)-1 oxo-4 [(pipérazinyl-1)-3 azétidinyl-1]-8 dihydro-1,4 benzo[b]naphtyridine-1,8 carboxylique-3 ;
- acide {[(amino-2 éthyl)-4 phényl] amino-3 azétidinyl-1}-8 difluoro-7,9 (fluoro-2 éthyl)-1 oxo-4 dihydro-1,4 benzo[b]naphtyridine-1,8 carboxylique-3 ;
- acide (amino-3 phényl-3 azétidinyl-1)-8 difluoro-7,9 (fluoro-2 éthyl)-1 oxo-4 dihydro-1,4 benzo[b]naphtyridine-1,8 carboxylique-3 ;
- acide (amino-3 méthyl-3 azétidinyl-1)-8 difluoro-7,9 (fluoro-2 éthyl)-1 oxo-4 dihydro-1,4 benzo[b]naphtyridine-1,8 carboxylique-3 ;
- acide (amino-3 diméthylamino-2 azétidinyl-1)-8 difluoro-7,9 (fluoro-2 éthyl)-1 oxo-4 dihydro-1,4 benzo[b]naphtyridine-1,8 carboxylique-3 ;
- acide fluoro-7 (méthylamino-3 azétidinyl-1)-8 oxo-4 t.butyl-1 dihydro-1,4 benzo[b]naphtyridine-1,8 carboxylique-3 ;
- acide (éthylamino-3 azétidinyl-1)-8 fluoro-7 oxo-4 t.butyl-1 dihydro-1,4 benzo[b]naphtyridine-1,8 carboxylique-3 ;
- acide [(amino-2 éthyl)amino-3 azétidinyl-1]-8 difluoro-7,9 oxo-4 t.butyl-1 dihydro-1,4 benzo[b]naphtyridine-1,8 carboxylique-3 ;
- acide difluoro-7,9 [(hydroxy-2 éthyl)amino-3 azétidinyl-1]-8 oxo-4 t.butyl-1 dihydro-1,4 benzo[b]naphtyridine-1,8 carboxylique-3 ;
- acide (cyclopropylamino-3 azétidinyl-1)-8 difluoro-7,9 oxo-4 t.butyl-1 dihydro-1,4 benzo[b]naphtyridine-1,8 carboxylique-3 ;
- acide difluoro-7,9 oxo-4 [(pipérazinyl-1)-3 azétidin-yl-1]-8 t.butyl-1 dihydro-1,4 benzo[b]naphtyridine-1,8 carboxylique-3 ;
- acide {[(amino-2 éthyl)-4 phényl] amino-3 azétidinyl-1}-8 difluoro-7,oxo-4 t.butyl-1 dihydro-1,4 benzo[b]naphtyridine-1,8 carboxylique-3 ;
- acide (amino-3 phényl-3 azétidinyl-1)-8 difluoro-7,9 oxo-4 t.butyl-1 dihydro-1,4 benzo[b]naphtyridine-1,8 carboxylique-3 ;
- acide (amino-3 méthyl-3 azétidinyl-1)-8 difluoro-7,9 oxo-4 t.butyl-1 dihydro-1,4 benzo[b]naphtyridine-1,8 carboxylique-3 ;
- acide (amino-3 diméthylamino-2 azétidinyl-1)-8 difluoro-7,9 oxo-4 t.butyl-1 dihydro-1,4 benzo[b]naphtyridine-1,8 carboxylique-3 ;

La présente invention concerne également les compositions pharmaceutiques utilisables en médecine humaine ou vétérinaire qui contiennent comme produit actif au moins un produit de formule générale (I) à l'état pur (sous forme libre ou sous forme de sel) ou sous forme d'une association avec un ou plusieurs diluants ou adjuvants compatibles et pharmaceutiquement acceptables. Ces compositions peuvent être utilisées par voie orale, parentérale ou rectale.

Comme compositions solides pour administration orale, peuvent être utilisés des comprimés, des gélules, des pilules, des poudres ou des granulés. Dans ces compositions, le produit actif selon l'invention peut être mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale, peuvent être des solutions stériles aqueuses ou non aqueuses, des suspensions ou des émulsions. Comme solvant ou véhicule, on peut employer le propylèneglycol, un polyéthylèneglycol, des huiles végétales, en particulier l'huile d'olive, et des esters organiques injectables, par exemple l'oléate d'éthyle. Ces compositions peuvent également contenir des adjuvants, en particulier des agents mouillants, émulsifiants, dispersants ou isotonisants. La stérilisation peut se faire de plusieurs façons, par exemple à l'aide d'un filtre bactériologique, en incorporant à la composition des agents stérilisants, par irradiation ou par chauffage. Elles peuvent également être préparées sous forme de compositions solides stériles qui seront dissoutes au moment de l'emploi dans de l'eau stérile ou tout autre milieu stérile injectable.

Les compositions pour administration rectale sont les suppositoires ou les capsules rectales qui peuvent contenir, outre le produit actif, des excipients tels que le beurre de cacao ou la suppo-cire.

En thérapeutique humaine ou vétérinaire, les compositions selon l'invention sont particulièrement utiles dans le traitement des infections d'origine bactérienne.

D'une façon générale, le médecin déterminera la posologie qu'il estime la plus appropriée en fonction de l'âge, du poids, du degré de l'infection et des autres facteurs propres au sujet à traiter. Généralement, les doses sont comprises entre 0,2 et 1 g de produit actif deux fois par jour, par voie orale ou parentérale pour un adulte.

L'exemple suivant, donné à titre non limitatif, illustre une composition selon l'invention :

### Exemple

On prépare selon les techniques habituelles des comprimés dosés à 250 mg de produit actif, ayant la composition suivante :
- acide (amino-3 azétidinyl-1)-6 cyclopropyl-1 fluoro-7 méthyl-1 oxo-4 dihydro-1,4 benzo[b]naphtyridine-1,8 carboxylique-3 250 mg
- amidon 50 mg
- lactose 35 mg
- talc 15 mg

Les produits de formule générale (I) sont également intéressants dans le domaine de l'agrochimie pour les traitements antibactériens des plantes et des végétaux. Il est entendu que les compositions à usage agrochimique renfermant un produit de formule générale (I) entrent aussi dans le cadre de la présente invention.

Par ailleurs, les produits de formule générale (I) peuvent également être utilisés comme agents de conservation ou de désinfection des matières organiques ou minérales. Notamment dans l'industrie des colorants, de matières grasses, du papier, du bois, des polymères ou encore dans l'industrie textile, l'industrie alimentaire ou le traitement des eaux. Il est également entendu que les compositions renfermant un produit de formule générale (I) à l'état pur ou sous forme d'association avec des diluants ou adjuvants compatibles, entrent aussi dans le cadre de la présente invention.

## Revendications

1. Un dérivé de la benzo[b]naphtyridine-1,8 caractérisé en ce qu'il répond à la formule générale : 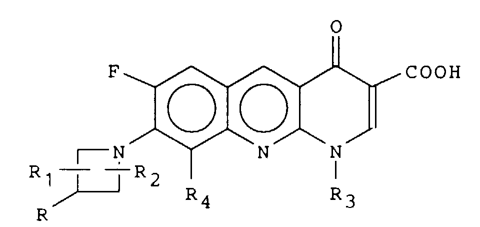 dans laquelle,
- R représente un atome d'hydrogène ou un radical hydroxy, amino, alcoylamino dont la partie alcoyle est éventuellement substituée par un radical amino ou hydroxy ou représente un radical dialcoylamino dont les parties alcoyle peuvent éventuellement former avec l'atome d'azote auquel elles sont rattachées, un hétérocycle à 5 ou 6 chaînons contenant éventuellement un autre hétéroatome choisi parmi l'azote, l'oxygène ou le soufre, ou représente un radical cycloalcoylamino contenant 3 à 6 chaînons, ou un radical alcanoylamino, N-alcoyl N-alcanoyl amino ou aminoalcoylphénylamino,
- R₁ et R₂ identiques ou différents sont respectivement situés en position 2 et 3 et représentent des atomes d'hydrogène, des radicaux alcoyle, alcènyle contenant 2 à 4 atomes de carbone, phényle, phényle substitué par un atome d'halogène, ou par un radical alcoyle, alcoyloxy, hydroxy, nitro, amino, alcoylamino, dialcoylamino ou halogénoalcoyle, ou bien R₁ et R₂ sont situés en position 2 et représentent des radicaux alcoyle,
- R₃ représente un atome d'hydrogène ou un radical alcoyle, fluoroalcoyle, carboxyalcoyle, cycloalcoyle contenant 3 à 6 atomes de carbone, fluorophényle, difluorophényle, alcoyloxy ou alcoylamino, et
- R₄ représente un atome d'hydrogène ou un atome de fluor,
les radicaux alcoyle et alcanoyle cités ci-dessus étant droits ou ramifiés et contennant 1 à 4 atomes de carbone,
sous ses formes stéréoisomères ou leurs mélanges ainsi que ses sels métalliques, ses sels d'addition avec les bases azotées, ses sels d'addition avec les acides et ses formes hydratées.

2. Procédé de préparation d'un dérivé de la benzo[b]naphtyridine-1,8 selon la revendication 1, caractérisé en ce que l'on fait agir un dérivé de l'azétidine de formule générale : 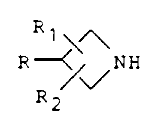 dans laquelle R, R₁ et R₂ sont définis comme dans la revendication 1, sur une benzo[b]naphtyridine-1,8 de formule générale : 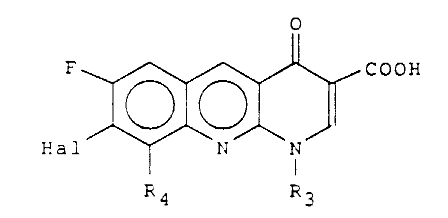 dans laquelle R₃ et R₄ sont définis comme dans la revendication 1 et Hal est un atome de fluor, de chlore ou de brome, si R₄ est un atome d'hydrogène, ou Hal et R₄ sont simultanément des atomes de fluor, puis éventuellement transforme le produit obtenu en un sel.

3. Procédé de préparation d'un dérivé de la benzo[b]naphtyridine-1,8 selon la revendication 1, caractérisé en ce que l'on transforme l'ester de formule générale : 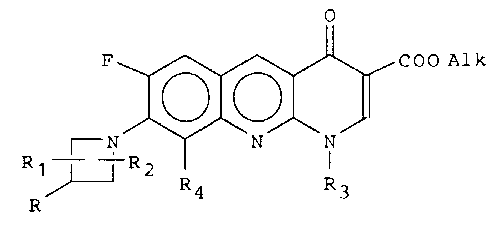 dans laquelle R₁, R₂ et R₄ sont définis comme dans la revendication 1, R est défini comme dans la revendication 1 ou représente un radical amino protégé, R₃ est défini comme dans la revendication 1 ou représente un radical alcoylamino protégé et Alk est un radical alcoyle droit ou ramifié contenant 1 à 4 atomes de carbone, par toute méthode connue pour obtenir un acide à partir d'un ester, sans toucher au reste de la molécule, puis, le cas échéant, élimine le groupement protecteur du radical alcoylamino, et/ou éventuellement prépare le sel du dérivé de benzo[b]naphtyridine obtenu.

4. Composition caractérisée en ce qu'elle contient au moins un dérivé selon la revendication 1, à l'état pur ou en association avec un ou plusieurs diluants ou adjuvants compatibles.

## Patentansprüche

1. 1,8-Benzo[b]naphthyridinderivat, dadurch gekennzeichnet, daß es der allgemeinen Formel 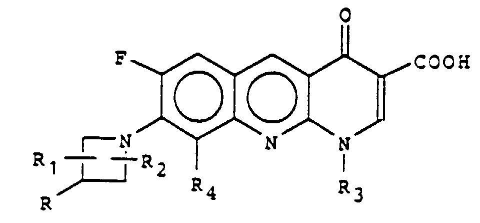 entspricht, worin
- R ein Wasserstoffatom oder einen Hydroxy-, Amino- oder Alkylaminorest, dessen Alkylteil gegebenenfalls durch einen Amino- oder Hydroxyrest substituiert ist, bedeutet oder für einen Dialkylaminorest steht, dessen Alkylteile gegebenenfalls mit dem Stickstoffatom, an das sie gebunden sind, einen Heterozyklus mit 5 oder 6 Gliedern bilden können, der gegebenenfalls ein weiteres Heteroatom, ausgewählt unter Stickstoff, Sauerstoff oder Schwefel, enthält, oder einen Cycloalkylaminorest mit 3 bis 6 Gliedern oder einen Alkanoylamino-, N- Alkyl-N-alkanoylamino- oder Aminoalkylphenylaminorest bedeutet,
- R₁ und R₂, identisch oder verschieden, in 2- bzw. 3Stellung vorliegen und Wasserstoffatome, Alkyl- oder Alkenylreste mit 2 bis 4 Kohlenstoffatomen, Phenyl, Phenyl, substituiert durch ein Halogenatom oder durch eine Alkyl-, Alkoxy-, Hydroxy-, Nitro-, Amino-, Alkylamino-, Dialkylamino- oder Halogenoalkylgruppe, bedeuten oder R₁ und R₂ sich in 2-Stellung befinden und Alkylreste darstellen,
- R₃ ein Wasserstoffatom oder einen Alkyl-, Fluoralkyl-, Carboxyalkyl-, Cycloalkyl- mit 3 bis 6 Kohlenstoffatomen, Fluorphenyl-, Difluorphenyl-, Alkoxy- oder Alkylaminorest bedeuten und
- R₄ ein Wasserstoffatom oder ein Fluoratom darstellt,
wobei die vorstehend genannten Alkyl- und Alkanoylreste geradkettig oder verzweigt sind und 1 bis 4 Kohlenstoffatome enthalten,
in ihren stereoisomeren Formen oder ihren Gemischen sowie die Metallsalze, die Additionssalze mit Stickstoffbasen, die Additionssalze mit Säuren und ihre hydratisierten Formen.

2. Verfahren zur Herstellung eines 1,8-Benzo[b]naphthyridinderivats gemäß Anspruch 1, dadurch gekennzeichnet, daß man ein Azetidinderivat der allgemeinen Formel 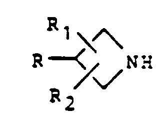 worin R, R₁ und R₂ wie in Anspruch 1 definiert sind, mit einem 1,8-Benzo[b]naphthyridin der allgemeinen Formel 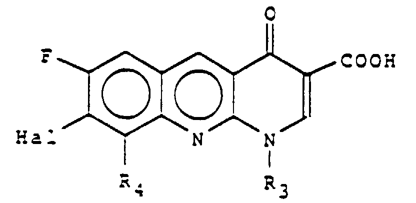 worin R₃ und R₄ wie in Anspruch 1 definiert sind und Hal für ein Fluor-, Chlor- oder Bromatom steht, wenn R₄ ein Wasserstoffatom bedeutet, oder Hal und R₄ gleichzeitig Fluoratome sind, umsetzt und danach gegebenenfalls das erhaltene Produkt in ein Salz überführt.

3. Verfahren zur Herstellung eines 1,8-Benzo[b]naphthyridinderivats gemäß Anspruch 1, dadurch gekennzeichnet, daß man den Ester der allgemeinen Formel 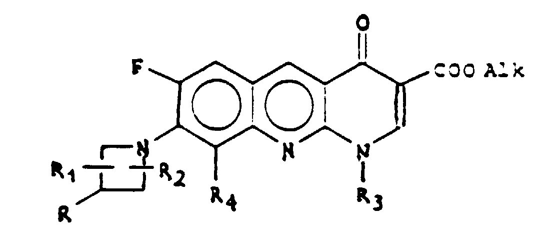 worin R₁, R₂ und R₄ wie in Anspruch 1 definiert sind, R wie in Anspruch 1 definiert ist oder eine geschützte Aminogruppe bedeutet, R₃ wie in Anspruch 1 definiert ist oder eine geschützte Alkylaminogruppe bedeutet und Alk einen geradkettigen oder verzweigten Alkylrest mit 1 bis 4 Kohlenstoffatomen wiedergibt, nach irgendeiner bekannten Methode für die Erzielung einer Säure aus einem Ester ohne Beeinträchtigung des Rests des Moleküls umwandelt und danach gegebenenfalls die Schutzgruppe des Alkylaminorests eliminiert und/oder gegebenenfalls das Salz des erhaltenen Benzo[b]naphthyridinderivats herstellt.

4. Zusammensetzung, dadurch gekennzeichnet, daß sie zumindest ein Derivat gemäß Anspruch 1 in reiner Form oder in Assoziation mit einem oder mehreren verträglichen Verdünnungsmitteln oder Adjuvantien enthält.

## Claims

1. A benzo[b] [1,8]naphthyridine derivative, characterized in that it corresponds to the general formula: 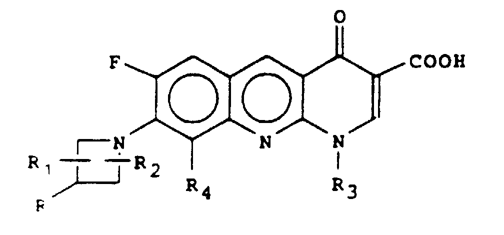 in which,
- R represents a hydrogen atom or a hydroxyl, amino or alkylamino radical, the alkyl part of the latter being optionally substituted by an amino or hydroxyl radical, or represents a dialkylamino radical, the alkyl parts of which can optionally form, with the nitrogen atom to which they are attached, a 5- or 6-membered heterocycle optionally containing another heteroatom chosen from nitrogen, oxygen or sulphur, or represents a cycloalkylamino radical containing 3 to 6 chain members, or an alkanoylamino, N-alkyl-N-alkanoylamino or aminoalkylphenylamino radical,
- R₁ and R₂, which are identical or different, are respectively situated at the 2- and 3-positions and represent hydrogen atoms or radicals of the type alkyl, alkenyl containing 2 to 4 carbon atoms, phenyl, phenyl substituted by a halogen atom, or by a radical of the type alkyl, alkyloxy, hydroxyl, nitro, amino, alkylamino, dialkylamino or haloalkyl, or alternatively R₁ and R₂ are situated at the 2-position and represent alkyl radicals,
- R₃ represents a hydrogen atom or an alkyl, fluoroalkyl, carboxyalkyl, cycloalkyl containing 3 to 6 carbon atoms, fluorophenyl, difluorophenyl, alkyloxy or alkylamino radical, and
- R₄ represents a hydrogen atom or a fluorine atom,
the alkyl and alkanoyl radicals mentioned above being straight or branched and containing 1 to 4 carbon atoms,
in its stereoisomeric forms or their mixtures, as well as its metal salts, its addition salts with nitrogenous bases, its addition salts with acids and its hydrated forms.

2. Process for the preparation of a benzo[b] [1,8]-naphthyridine derivative according to Claim 1, characterized in that an azetidine derivative of general formula: 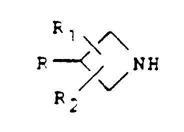 in which R, R₁ and R₂ are defined as in Claim 1, is reacted with a benzo[b][1,8]naphthyridine of general formula: 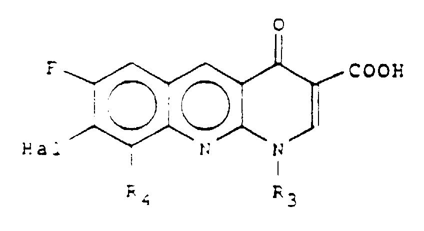 in which R₃ and R₄ are defined as in Claim 1 and Hal is a fluorine, chlorine or bromine atom, if R₄ is a hydrogen atom, or Hal and R₄ are simultaneously fluorine atoms, and then the product obtained is optionally converted to a salt.

3. Process for the preparation of a benzo[b][1,8]naphthyridine derivative according to Claim 1, characterized in that the ester of general formula: 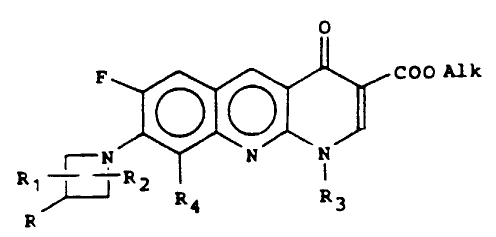 in which R₁, R₂ and R₄ are defined as in Claim 1, R is defined as in Claim 1 or represents a protected amino radical, R₃ is defined as in Claim 1 or represents a protected alkylamino radical and Alk is a straight or branched alkyl radical containing 1 to 4 carbon atoms, is converted, by any method known for obtaining an acid from an ester, without affecting the remainder of the molecule, and then, if appropriate, the protective group for the alkylamino radical is removed and/or, optionally, the salt of the benzo[b]naphthyridine derivative obtained is prepared.

4. Composition, characterized in that it contains at least one derivative according to Claim 1, in the pure state or in combination with one or more compatible diluents or adjuvants.
